(19) Europäisches Patentamt
European Patent Office
Office européen des brevets

(11) EP 3 895 171 B1

(12) **EUROPEAN PATENT SPECIFICATION**

(45) Date of publication and mention of the grant of the patent:
**23.07.2025 Bulletin 2025/30**

(21) Application number: **19832518.5**

(22) Date of filing: **10.12.2019**

(51) International Patent Classification (IPC):
***G16B 30/20*** *(2019.01)* ***G16B 40/20*** *(2019.01)*

(52) Cooperative Patent Classification (CPC):
**G16B 30/20; G16B 40/20**

(86) International application number:
**PCT/US2019/065540**

(87) International publication number:
**WO 2020/123552 (18.06.2020 Gazette 2020/25)**

(54) **DEEP BASECALLER FOR SANGER SEQUENCING**

BASENZUWEISUNG FÜR DIE SANGER-SEQUENZIERUNG MITTELS DEEP LEARNING

APPEL DE BASE POUR LE SÉQUENÇAGE DE SANGER À L'AIDE DE L'APPRENTISSAGE PROFOND

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.12.2018 US 201862777429 P**

(43) Date of publication of application:
**20.10.2021 Bulletin 2021/42**

(73) Proprietor: **Life Technologies Corporation**
**Carlsbad, CA 92008 (US)**

(72) Inventors:
- **CHU, Yong**
  **Carlsbad, California 92008 (US)**
- **SCHNEIDER, Stephanie**
  **Carlsbad, California 92008 (US)**
- **SCHAEFFER, Rylan**
  **Carlsbad, California 92008 (US)**
- **WOO, David**
  **Carlsbad, California 92008 (US)**

(74) Representative: **Hoffmann Eitle**
**Patent- und Rechtsanwälte PartmbB**
**Arabellastraße 30**
**81925 München (DE)**

(56) References cited:
**WO-A1-2019/147904**

- **OMNIYAH G. MOHAMMED ET AL: "Novel algorithms for accurate DNA base-calling", JOURNAL OF BIOMEDICAL SCIENCE AND ENGINEERING, vol. 06, no. 02, 1 January 2013 (2013-01-01), pages 165 - 174, XP055576739, ISSN: 1937-6871, DOI: 10.4236/jbise.2013.62020**
- **HAOTIAN TENG ET AL: "Chiron: translating nanopore raw signal directly into nucleotide sequence using deep learning", GIGASCIENCE, vol. 7, no. 5, 10 April 2018 (2018-04-10), XP055492410, DOI: 10.1093/gigascience/giy037**
- **VLADIMÍR BOZA ET AL: "DeepNano: Deep recurrent neural networks for base calling in MinION nanopore reads", PLOS ONE, vol. 12, no. 6, 5 June 2017 (2017-06-05), pages e0178751, XP055669713, DOI: 10.1371/journal.pone.0178751**
- **RATKOVIC M: "Deep Learning Model for Base Calling of MinION Nanopore Reads", INTERNET CITATION, 1 June 2017 (2017-06-01), pages 1 - 48, XP002789430, Retrieved from the Internet <URL:https://bib.irb.hr/datoteka/884158.Diplomski_2017_Marko_Ratkovi.pdf> [retrieved on 20190301]**
- **TIANWEI YUE ET AL: "Deep Learning for Genomics: A Concise Overview", ARXIV.ORG, CORNELL UNIVERSITY LIBRARY, 201 OLIN LIBRARY CORNELL UNIVERSITY ITHACA, NY 14853, 2 February 2018 (2018-02-02), XP080857057**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MATIUR RAHMAN MINAR ET AL: "Recent Advances in Deep Learning: An Overview", ARXIV.ORG, CORNELL UNIVERSITY LIBRARY, 201 OLIN LIBRARY CORNELL UNIVERSITY ITHACA, NY 14853, 21 July 2018 (2018-07-21), XP081250437, DOI: 10.13140/RG.2.2.24831.10403

## Description

## FIELD

The present disclosure relates generally to systems, devices, and methods for basecalling, and more specifically to systems, devices, and methods for basecalling using deep machine learning in Sanger sequencing analysis.

## BACKGROUND

Sanger Sequencing with capillary electrophoresis (CE) genetic analyzers is the gold-standard DNA sequencing technology, which provides a high degree of accuracy, longread capabilities, and the flexibility to support a diverse range of applications in many research areas. The accuracies of basecalls and quality values (QVs) for Sanger Sequencing on CE genetic analyzers are essential for successful sequencing projects. A legacy basecaller was developed to provide a complete and integrated basecalling solution to support sequencing platforms and applications. It was originally engineered to basecall long plasmid clones (pure bases) and then extended later to basecall mixed base data to support variant identification.

However, obvious mixed bases are occasionally called as pure bases even with high predicted QVs, and false positives in which pure bases are incorrectly called as mixed bases, also occur relatively frequently due to sequencing artefacts such as dye blobs, n-1 peaks due to polymerase slippage and primer impurities, mobility shifts, etc. Clearly, the basecalling and QV accuracy for mixed bases need to be improved to support sequencing applications for identifying variants such as Single Nucleotide Polymorphisms (SNPs) and heterozygous insertion deletion variants (het indels). The basecalling accuracy of legacy basecallers at 5' and 3' ends is also relatively low due to mobility shifts and low resolution at 5' and 3' ends. The legacy basecaller also struggles to basecall amplicons shorter than 150 base pairs (bps) in length, particularly shorter than 100 bps, failing to estimate average peak spacing, average peak width, spacing curve, and/or width curve, sometimes resulting in increased error rate.

Therefore, improved basecalling accuracy for mixed bases and 5' and 3' ends is very desirable so that basecalling algorithms can deliver higher fidelity of Sanger Sequencing data, improve variant identification, increase read length, and also save sequencing costs for sequencing applications.

Denaturing capillary electrophoresis is well known to those of ordinary skill in the art. In overview, a nucleic acid sample is injected at the inlet end of the capillary, into a denaturing separation medium in the capillary, and an electric field is applied to the capillary ends. The different nucleic acid components in a sample, e.g., a polymerase chain reaction (PCR) mixture or other sample, migrate to the detector point with different velocities due to differences in their electrophoretic properties. Consequently, they reach the detector (usually an ultraviolet (UV) or fluorescence detector) at different times. Results present as a series of detected peaks, where each peak represents ideally one nucleic acid component or species of the sample. Peak area and/or peak height indicate the initial concentration of the component in the mixture.

The magnitude of any given peak, including an artifact peak, is most often determined optically on the basis of either UV absorption by nucleic acids, e.g., DNA, or by fluorescence emission from one or more labels associated with the nucleic acid. UV and fluorescence detectors applicable to nucleic acid CE detection are well known in the art.

CE capillaries themselves are frequently quartz, although other materials known to those of skill in the art can be used. There are a number of CE systems available commercially, having both single and multiple-capillary capabilities. The methods described herein are applicable to any device or system for denaturing CE of nucleic acid samples.

Because the charge-to-frictional drag ratio is the same for different sized polynucleotides in free solution, electrophoretic separation requires the presence of a sieving (i.e., separation) medium. Applicable CE separation matrices are compatible with the presence of denaturing agents necessary for denaturing nucleic acid CE, a common example of which is 8M urea.

The ability to decipher the genetic code of different species would lead to significant future scientific achievements in important areas, including medicine and agriculture. In OMNIYAH G. MOHAMMED ET AL: "Novel algorithms for accurate DNA base-calling",

> JOURNAL OF BIOMEDICAL SCIENCE AND ENGINEERING,
> vol. 06, no. 02, 1 January 2013 (2013-01-01), pages 165-174,
> XP055576739,
> ISSN: 1937-6871, DOI: 10.4236/jbise.2013.62020
> a pattern recognition technique was adopted to minimize the inaccuracy in DNA basecalling.
> HAOTIAN TENG ET AL: "Chiron: translating nanopore raw signal directly into nucleotide sequence using deep learning",
> GIGASCIENCE,
> vol. 7, no. 5, 10 April 2018 (2018-04-10), XP055492410,
> DOI: 10.1093/gigascience/giy037
> reports the first deep learning model to achieve end-to-end basecalling and directly translate the raw signal to DNA sequence without the error-prone segmentation step.
> VLADIMÍR BOZA ET AL: "DeepNano: Deep recurrent neural networks for base calling in MinION nanopore reads",

PLOS ONE,
vol. 12, no. 6, 5 June 2017 (2017-06-05), page e0178751, XP055669713, DOI: 10.137/journal.pone.0178751
presents an open-source DNA base caller based on deep recurrent neural networks and shows that the accuracy of base calling is much dependent on the underlying software an can be improved by considering modern machine learning methods.

In recent years, deep learning methods significantly improved the state-of-the-art in multiple domains such as computer vision, speech recognition, and natural language processing. In RATKOVIC M: "RATKOVIC MDeep learning Model for Base Calling of MinION Nanopore Reads",

INTERNET CITATION, 1 June 2017 (2017-06-01), pages 1-48,
XP002789430,
application of deep learning in the field of Bioinformatics is presented for analysis of DNA sequencing data.

## SUMMARY

Systems and methods are described for use in basecalling applications, for example in basecalling systems based on microfluidic separations (in which separation is performed through micro-channels etched into or onto glass, silicon or other substrate), or separation through capillary electrophoresis using single or multiple cylindrical capillary tubes. A neural network control system and a process control method is defined in the appended claims.

## BRIEF DESCRIPTION OF THE DRAWINGS

To easily identify the discussion of any particular element or act, the most significant digit or digits in a reference number refer to the figure number in which that element is first introduced.

FIG. 1 illustrates a CE device 100 in accordance with one embodiment.
FIG. 2 illustrates a CE system 200 in accordance with one embodiment.
FIG. 3 illustrates a CE process 300 in accordance with one embodiment.
FIG. 4 illustrates a CE process 400 in accordance with one embodiment.
FIG. 5 illustrates a basic deep neural network 500 in accordance with one embodiment.
FIG. 6 illustrates an artificial neuron 600 in accordance with one embodiment.
FIG. 7 illustrates a recurrent neural network 700 in accordance with one embodiment.
FIG. 8 illustrates a bidirectional recurrent neural network 800 in accordance with one embodiment.
FIG. 9 illustrates a long short-term memory 900 in accordance with one embodiment.
FIG. 10 illustrates a basecaller system 1000 in accordance with one embodiment.
FIG. 11 illustrates a scan label model training method 1100 in accordance with one embodiment.
FIG. 12 illustrates a QV model training method 1200 in accordance with one embodiment.
FIG. 13 is an example block diagram of a computing device 1300 that may incorporate embodiments of the present invention.

## DETAILED DESCRIPTION

Terminology used herein should be accorded its ordinary meaning the arts unless otherwise indicated expressly or by context.

"Quality values" in this context refers to an estimate (or prediction) of the likelihood that a given basecall is in error. Typically, the quality value is scaled following the convention established by the Phred program: QV = -10 log10(Pe), where Pe stands for the estimated probability that the call is in error. Quality values are a measure of the certainty of the base calling and consensus-calling algorithms. Higher values correspond to lower chance of algorithm error. Sample quality values refer to the per base quality values for a sample, and consensus quality values are per-consensus quality values.

"Sigmoid function" in this context refers to a function of the form f(x)=1/(exp(-x)). The sigmoid function is used as an activation function in artificial neural networks. It has the property of mapping a wide range of input values to the range 0-1, or sometimes -1 to 1.

"Capillary electrophoresis genetic analyzer" in this context refers to instrument that applies an electrical field to a capillary loaded with a sample so that the negatively charged DNA fragments move toward the positive electrode. The speed at which a DNA fragment moves through the medium is inversely proportional to its molecular weight. This process of electrophoresis can separate the extension products by size at a resolution of one base.

"Image signal" in this context refers to an intensity reading of fluorescence from one of the dyes used to identify bases during a data run. Signal strength numbers are shown in the Annotation view of the sample file.

"Exemplary commercial CE devices" in this context refers to include the Applied Biosystems, Inc. (ABI) genetic analyzer models 310 (single capillary), 3130 (4 capillary), 3130xL (16 capillary), 3500 (8 capillary), 3500xL (24 capillary), 3730 (48 capillary), and 3730xL (96 capillary), the Agilent 7100 device, Prince Technologies, Inc.'s PrinCE™ Capillary Electrophoresis System, Lumex, Inc.'s Capel-105™ CE system, and Beckman Coulter's P/ACE™ MDQ systems, among others.

"Base pair" in this context refers to complementary nucleotide in a DNA sequence. Thymine (T) is complementary to adenine (A) and guanine (G) is complementary to cytosine (C).

"ReLU" in this context refers to a rectifier function, an activation function defined as the positive part of ints input. It is also known as a ramp function and is analogous to half-wave rectification in electrical signal theory. ReLU is a popular activation function in deep neural networks.

"Heterozygous insertion deletion variant" in this context refers to see single nucleotide polymorphism

"Mobility shift" in this context refers to electrophoretic mobility changes imposed by the presence of different fluorescent dye molecules associated with differently labeled reaction extension products.

"Variant" in this context refers to bases where the consensus sequence differs from the reference sequence that is provided.

"Polymerase slippage" in this context refers to is a form of mutation that leads to either a trinucleotide or dinucleotide expansion or contraction during DNA replication. A slippage event normally occurs when a sequence of repetitive nucleotides (tandem repeats) are found at the site of replication. Tandem repeats are unstable regions of the genome where frequent insertions and deletions of nucleotides can take place.

"Amplicon" in this context refers to the product of a PCR reaction. Typically, an amplicon is a short piece of DNA.

"Basecall" in this context refers to assigning a nucleotide base to each peak (A, C, G, T, or N) of the fluorescence signal.

"Raw data" in this context refers to a multicolor graph displaying the fluorescence intensity (signal) collected for each of the four fluorescent dyes.

"Base spacing" in this context refers to the number of data points from one peak to the next. A negative spacing value or a spacing value shown in red indicates a problem with your samples, and/or the analysis parameters.

"Separation or sieving media" in this context refers to include gels, however nongel liquid polymers such as linear polyacrylamide, hydroxyalkyl cellulose (HEC), agarose, and cellulose acetate, and the like can be used. Other separation media that can be used for capillary electrophoresis include, but are not limited to, water soluble polymers such as poly(N,N'-dimethyl acrylamide)(PDMA), polyethylene glycol (PEG), poly(vinylpyrrolidone) (PVP), polyethylene oxide, polysaccharides and pluronic polyols; various polyvinyl alcohol (PVAL)-related polymers, polyether-water mixture, lyotropic polymer liquid crystals, among others.

"Adam optimizer" in this context refers to an optimization algorithm that can used instead of the classical stochastic gradient descent procedure to update network weights iterative based in training data. Stochastic gradient descent maintains a single learning rate (termed alpha) for all weight updates and the learning rate does not change during training. A learning rate is maintained for each network weight (parameter) and separately adapted as learning unfolds. Adam as combining the advantages of two other extensions of stochastic gradient descent. Specifically, Adaptive Gradient Algorithm (AdaGrad) that maintains a per-parameter learning rate that improves performance on problems with sparse gradients (e.g. natural language and computer vision problems), and Root Mean Square Propagation (RMSProp) that also maintains per-parameter learning rates that are adapted based on the average of recent magnitudes of the gradients for the weight (e.g. how quickly it is changing). This means the algorithm does well on online and non-stationary problems (e.g. noisy). Adam realizes the benefits of both AdaGrad and RMSProp. Instead of adapting the parameter learning rates based on the average first moment (the mean) as in RMSProp, Adam also makes use of the average of the second moments of the gradients (the uncentered variance). Specifically, the algorithm calculates an exponential moving average of the gradient and the squared gradient, and the parameters beta1 and beta2 control the decay rates of these moving averages. The initial value of the moving averages and beta1 and beta2 values close to 1.0 (recommended) result in a bias of moment estimates towards zero. This bias is overcome by first calculating the biased estimates before then calculating biascorrected estimates.

"Hyperbolic tangent function" in this context refers to a function of the form tanh(x)=sinh(x)/cosh(x). The tanh function is a popular activation function in artificial neural networks. Like the sigmoid, the tanh function is also sigmoidal ("s"-shaped), but instead outputs values that range (-1, 1). Thus, strongly negative inputs to the tanh will map to negative outputs. Additionally, only zero-valued inputs are mapped to near-zero outputs. These properties make the network less likely to get "stuck" during training.

"Relative fluoresce unit" in this context refers to measurements in electrophoresis methods, such as for DNA analysis. A "relative fluorescence unit" is a unit of measurement used in analysis which employs fluorescence detection.

"CTC loss function" in this context refers to connectionist temporal classification, a type of neural network output and associated scoring function, for training recurrent neural networks (RNNs) such as LSTM networks to tackle sequence problems where the timing is variable. A CTC network has a continuous output (e.g. Softmax), which is fitted through training to model the probability of a label. CTC does not attempt to learn boundaries and timings: Label sequences are considered equivalent if they differ only in alignment, ignoring blanks. Equivalent label sequences can occur in many ways - which makes scoring a non-trivial task. Fortunately, there is an efficient forward-backward algorithm for that. CTC scores can then be used with the back-propagation algorithm to update the neural network weights. Alternative approaches to a CTC-fitted neural network include a hidden Markov model (HMM).

"Polymerase" in this context refers to an enzyme that catalyzes polymerization. DNA and RNA polymerases build single□ stranded DNA or RNA (respectively) from free nucleotides, using another single□ stranded DNA or RNA as the template.

"Sample data" in this context refers to the output of a single lane or capillary on a sequencing instrument. Sample data is entered into Sequencing Analysis, SeqScape, and other sequencing analysis software.

"Plasmid" in this context refers to a genetic structure in a cell that can replicate independently of the chromosomes, typically a small circular DNA strand in the cytoplasm of a bacterium or protozoan. Plasmids are much used in the laboratory manipulation of genes.

"Beam search" in this context refers to a heuristic search algorithm that explores a graph by expanding the most promising node in a limited set. Beam search is an optimization of best-first search that reduces its memory requirements. Best-first search is a graph search which orders all partial solutions (states) according to some heuristic. But in beam search, only a predetermined number of best partial solutions are kept as candidates. It is thus a greedy algorithm. Beam search uses breadth-first search to build its search tree. At each level of the tree, it generates all successors of the states at the current level, sorting them in increasing order of heuristic cost. However, it only stores a predetermined number, $\beta$, of best states at each level (called the beam width). Only those states are expanded next. The greater the beam width, the fewer states are pruned. With an infinite beam width, no states are pruned and beam search is identical to breadth-first search. The beam width bounds the memory required to perform the search. Since a goal state could potentially be pruned, beam search sacrifices completeness (the guarantee that an algorithm will terminate with a solution, if one exists). Beam search is not optimal (that is, there is no guarantee that it will find the best solution). In general, beam search returns the first solution found. Beam search for machine translation is a different case: once reaching the configured maximum search depth (i.e. translation length), the algorithm will evaluate the solutions found during search at various depths and return the best one (the one with the highest probability). The beam width can either be fixed or variable. One approach that uses a variable beam width starts with the width at a minimum. If no solution is found, the beam is widened and the procedure is repeated.

"Sanger Sequencer" in this context refers to a DNA sequencing process that takes advantage of the ability of DNA polymerase to incorporate 2',3'-dideoxynucleotidesnucleotide base analogs that lack the 3'-hydroxyl group essential in phosphodiester bond formation. Sanger dideoxy sequencing requires a DNA template, a sequencing primer, DNA polymerase, deoxynucleotides (dNTPs), dideoxynucleotides (ddNTPs), and reaction buffer. Four separate reactions are set up, each containing radioactively labeled nucleotides and either ddA, ddC, ddG, or ddT. The annealing, labeling, and termination steps are performed on separate heat blocks. DNA synthesis is performed at 37°C, the temperature at which DNA polymerase has the optimal enzyme activity. DNA polymerase adds a deoxynucleotide or the corresponding 2',3'-dideoxynucleotide at each step of chain extension. Whether a deoxynucleotide or a dideoxynucleotide is added depends on the relative concentration of both molecules. When a deoxynucleotide (A, C, G, or T) is added to the 3' end, chain extension can continue. However, when a dideoxynucleotide (ddA, ddC, ddG, or ddT) is added to the 3' end, chain extension 4 DNA Sequencing by Capillary terminates. Sanger dideoxy sequencing results in the formation of extension products of various lengths terminated with dideoxynucleotides at the 3' end.

"Single nucleotide polymorphism" in this context refers to a variation in a single base pair in a DNA sequence.

"Mixed base" in this context refers to one-base positions that contain 2, 3, or 4 bases. These bases are assigned the appropriate IUB code.

"Softmax function" in this context refers to a function of the form f(xi)=exp(xi)/sum(exp(x)) where the sum is taken over a set of x. Softmax is used at different layers (often at the output layer) of artificial neural networks to predict classifications for inputs to those layers. The Softmax function calculates the probabilities distribution of the event xi over 'n' different events. In general sense, this function calculates the probabilities of each target class over all possible target classes. The calculated probabilities are helpful for predicting that the target class is represented in the inputs. The main advantage of using Softmax is the output probabilities range. The range will 0 to 1, and the sum of all the probabilities will be equal to one. If the softmax function used for multi-classification model it returns the probabilities of each class and the target class will have the high probability. The formula computes the exponential (e-power) of the given input value and the sum of exponential values of all the values in the inputs. Then the ratio of the exponential of the input value and the sum of exponential values is the output of the softmax function.

"Noise" in this context refers to average background fluorescent intensity for each dye.

"Backpropagation" in this context refers to an algorithm used in artificial neural networks to calculate a gradient that is needed in the calculation of the weights to be used in the network. It is commonly used to train deep neural networks, a term referring to neural networks with more than one hidden layer. For backpropagation, the loss function calculates the difference between the network output and its expected output, after a case propagates through the network.

"Dequeue max finder" in this context refers to an algorithm utilizing a doubleended queue to determine a maximum value.

"Gated Recurrent Unit (GRU)" in this context refers to are a gating mechanism in recurrent neural networks. GRUs may exhibit better performance on smaller datasets than do LSTMs. They have fewer parameters than LSTM, as they lack an output gate. See https://en.wikipedia.org/wiki/Gated_recurrent_unit

"Pure base" in this context refers to assignment mode for a base caller, where the base caller determines an A, C, G, and T to a position instead of a variable.

"Primer" in this context refers to A short single strand of DNA that serves as the priming site for DNA polymerase in a PCR reaction.

"Loss function" in this context refers to also referred to as the cost function or error function (not to be confused with the Gauss error function), is a function that maps values of one or more variables onto a real number intuitively representing some "cost" associated with those values.

Referring to Figure 1, a CE device 100 in one embodiment comprises a voltage bias source 102, a capillary 104, a body 114, a detector 106, a sample injection port 108, a heater 110, and a separation media 112. A sample is injected into the sample injection port 108, which is maintained at an above-ambient temperature by the heater 110. Once injected the sample engages the separation media 112 and is split into component molecules. The components migrate through the capillary 104 under the influence of an electric field established by the voltage bias source 102, until they reach the detector 106.

Referencing Figure 2, a CE system 200 in one embodiment comprises a source buffer 218 initially comprising the fluorescently labeled sample 220, a capillary 222, a destination buffer 226, a power supply 228, a computing device 202 comprising a processor 208, memory 206 comprising basecaller algorithm 204, and a controller 212. The source buffer 218 is in fluid communication with the destination buffer 226 by way of the capillary 222. The power supply 228 applies voltage to the source buffer 218 and the destination buffer 226 generating a voltage bias through an anode 230 in the source buffer 218 and a cathode 232 in the destination buffer 226. The voltage applied by the power supply 228 is configured by a controller 212 operated by the computing device 202. The fluorescently labeled sample 220 near the source buffer 218 is pulled through the capillary 222 by the voltage gradient and optically labeled nucleotides of the DNA fragments within the sample are detected as they pass through an optical sensor 224. Differently sized DNA fragments within the fluorescently labeled sample 220 are pulled through the capillary at different times due to their size. The optical sensor 224 detects the fluorescent labels on the nucleotides as an image signal and communicates the image signal to the to the computing device 202. The computing device 202 aggregates the image signal as sample data and utilizes a basecaller algorithm 204 stored in memory 206 to operates a neural network 210 to transform the sample data into processed data and generate an electropherogram 216 to be shown in a display device 214.

Referencing Figure 3, a CE process 300 involves a computing device 312 communicating a configuration control 318 to a controller 308 to control the voltage applied by a power supply 306 to the buffers 302. After the prepared fluorescently labeled sample has been added to the source buffer, the controller 308 communicates an operation control 320 to the power supply 306 to apply a voltage 322 to the buffers creating a voltage bias/electrical gradient. The applied voltage cause the fluorescently labeled sample 324 to move through capillary 304 between the buffers 302 and pass by the optical sensor 310. The optical sensor 310 detects fluorescent labels on the nucleotides of the DNA fragments that pass through the capillary and communicates the image signal 326 to the computing device 312. The computing device 312 aggregates the image signal 326 to generate the sample data 328 that is communicated to a neural network 314 for further processing. The neural network 314 processes the sample data 328 (e.g., signal values) to generate processed data 330 (e.g., classes) that is communicated back to the computing device 312. The computing device 312 then generates a display control 332 to display an electropherogram in a display device 316.

Referencing Figure 4, a CE process 400 involves configuring a capillary electrophoresis instrument operating parameters to sequence at least one fluorescently labeled sample (block 402). The configuration of the instrument may include creating or importing a plate setting for running a series of samples and assigning labels to the plate samples to assist in the processing of the collected imaging data. The process may also include communicating configuration controls to a controller to start applying voltage at a predetermined time. In block 404, the CE process 400 loads the fluorescently labeled sample into the instrument. After the sample is loaded into the instrument, the instrument may transfer the sample from a plate well into the capillary tube and then position the capillary tube into the starting buffer at the beginning of the capillary electrophoresis process. In block 406, the CE process 400 begins the instrument run after the sample has been loaded into the capillary by applying a voltage to the buffer solutions positioned at opposite ends of the capillary, forming an electrical gradient to transport DNA fragments of the fluorescently labeled sample from the starting buffer to a destination buffer and traversing an optical sensor. In block 408, the CE process 400 detects the individual fluorescent signals on the nucleotides of the DNA fragments as they move towards the destination buffer through the optical sensor and communicates the image signal to the computing device. In block 410, the CE process 400 aggregates the image signal at the computing device from the optical sensor and generates sample data that corresponds to the fluorescent intensity of the nucleotides DNA fragments. In block 412, the CE process 400 processes the sample data through the utilization of a neural network to help identify the bases called in the DNA fragments at the particular time point. In block 414, the CE process 400 displays processed data through an electropherogram through a display device.

A basic deep neural network 500 is based on a collection of connected units or nodes called artificial neurons which loosely model the neurons in a biological brain. Each connection, like the synapses in a biological brain, can transmit a signal from one artificial neuron to another. An artificial neuron that receives a signal can process it and then signal additional artificial neurons connected to it.

In common implementations, the signal at a connection between artificial neurons is a real number, and the output of each artificial neuron is computed by some non-linear function (the activation function) of the sum of its inputs. The connections between artificial neurons are called 'edges' or axons. Artificial neurons and edges typically have a weight that adjusts as learning proceeds. The weight increases or decreases the strength of the signal at a connection. Artificial neurons may have a threshold (trigger threshold) such that the signal is only sent if the aggregate signal crosses that threshold. Typically, artificial neurons are aggregated into layers. Different layers may perform different kinds of transformations on their inputs. Signals travel from the first layer (the input layer 502), to the last layer (the output layer 506), possibly after traversing one or more intermediate layers, called hidden layers 504.

Referring to Figure 6, an artificial neuron 600 receiving inputs from predecessor neurons consists of the following components:

- inputs $x_i$;
- weights $w_i$ applied to the inputs;
- an optional threshold (b), which stays fixed unless changed by a learning function; and
- an activation function 602 that computes the output from the previous neuron inputs and threshold, if any.

An input neuron has no predecessor but serves as input interface for the whole network. Similarly, an output neuron has no successor and thus serves as output interface of the whole network.

The network includes connections, each connection transferring the output of a neuron in one layer to the input of a neuron in a next layer. Each connection carries an input x and is assigned a weight w.

The activation function 602 often has the form of a sum of products of the weighted values of the inputs of the predecessor neurons.

The learning rule is a rule or an algorithm which modifies the parameters of the neural network, in order for a given input to the network to produce a favored output. This learning process typically involves modifying the weights and thresholds of the neurons and connections within the network.

Figure 7 illustrates a recurrent neural network 700 (RNN). Variable x[t] is the input at stage t. For example, x[1] could be a one-hot vector corresponding to the second word of a sentence. Variable s[t] is the hidden state at stage t. It's the "memory" of the network. The variable s[t] is calculated based on the previous hidden state and the input at the current stage: s[t]=f(Ux[t] + Ws[t-1]). The activation function f usually is a nonlinearity such as tanh or ReLU. The input s(-1) , which is required to calculate the first hidden state, is typically initialized to all zeroes. Variable o[t] is the output at stage t. For example, to predict the next word in a sentence it would be a vector of probabilities across the vocabulary: o[t]=softmax(Vs[t]).

Figure 8 illustrates a bidirectional recurrent neural network 800 (BRNN). BRNNs are designed for situation where the output at a stage may not only depend on the previous inputs in the sequence, but also future elements. For example, to predict a missing word in a sequence a BRNN will consider both the left and the right context. BRNNs may be implemented as two RNNs in which the output Y is computed based on the hidden states S of both RNNs and the inputs X. In the bidirectional recurrent neural network 800 shown in Figure 8, each node A is typically itself a neural network. Deep BRNNs are similar to BRNNs, but have multiple layers per node A. In practice this enables a higher learning capacity but also requires more training data than for single layer networks.

Figure 9 illustrates a RNN architecture with long short-term memory 900 (LSTM).

All RNNs have the form of a chain of repeating nodes, each node being a neural network. In standard RNNs, this repeating node will have a structure such as a single layer with a tanh activation function. This is shown in the upper diagram. An LSTMs also has this chain like design, but the repeating node A has a different structure than for regular RNNs. Instead of having a single neural network layer, there are typically four, and the layers interact in a particular way.

In an LSTM each path carries an entire vector, from the output of one node to the inputs of others. The circled functions outside the dotted box represent pointwise operations, like vector addition, while the sigmoid and tanh boxes inside the dotted box are learned neural network layers. Lines merging denote concatenation, while a line forking denote values being copied and the copies going to different locations.

An important feature of LSTMs is the cell state Ct, the horizontal line running through the top of the long short-term memory 900 (lower diagram). The cell state is like a conveyor belt. It runs across the entire chain, with only some minor linear interactions. It's entirely possible for signals to flow along it unchanged. The LSTM has the ability to remove or add information to the cell state, carefully regulated by structures called gates. Gates are a way to optionally let information through a cell. They are typically formed using a sigmoid neural net layer and a pointwise multiplication operation.

The sigmoid layer outputs numbers between zero and one, describing how much of each component should be let through. A value of zero means "let nothing through," while a value of one means "let everything through". An LSTM has three of these sigmoid gates, to protect and control the cell state.

Referring to Figure 10, a basecaller system 1000 comprises an input segmenter 1002, a scan label model 1004, an assembler 1006, a decoder 1008, a quality value model 1010, and a sequencer 1012.

The input segmenter 1002 receives an input trace sequence, a window size, and a stride length. The input trace sequence may be a sequence of dye relative fluoresce units (RFUs) collected from a capillary electrophoresis (CE) instrument or raw spectrum data collected in the CE instruments directly. The input trace sequence comprises a number of scans. The window size determines the number of scans per input to the scan label model 1004. The stride length determines the number of windows, or inputs, to the scan label model 1004. The input segmenter 1002 utilizes the input trace sequence, a window size, and a stride length to generate the input scan windows to be sent to the scan label model 1004.

The scan label model 1004 receives the input scan windows and generates scan label probabilities for all scan windows. The scan label model 1004 may comprise one or more trained models. The models may be selected to be utilized to generate the scan label probabilities. The models may be BRNNs with one or more layers of LSTM or similar units, such as a GRU (Gated Recurrent Unit). The model may be have structure similar to that depicted in Figure 8, Figure 9 (deleted), and Figure 9. The model may further utilize a Softmax layer as the output layer of LSTM BRNN, which outputs the label probabilities for all scans in the input scan window. The scan label model 1004 may be trained in accordance with the process depicted in Figure 11. The scan label probabilities are then sent to the assembler 1006.

The assembler 1006 receives the scan label probabilities and assembles the label probabilities for all scan windows together to construct the label probabilities for the entire trace of the sequencing sample. The scan label probabilities for the assembled scan windows are then sent to the decoder 1008 and the quality value model 1010.

The decoder 1008 receives the scan label probabilities for the assembled scan windows. The decoder 1008 then decodes the scan label probabilities into basecalls for the input trace sequence. The decoder 1008 may utilize a prefix Beam search or other decoders on the assembled label probabilities to find the basecalls for the sequencing sample. The basecalls for the input trace sequence and the assembled scan windows are then sent to the sequencer 1012.

The quality value model 1010 receives the scan label probabilities for the assembled scan windows. The quality value model 1010 then generates an estimated basecalling error probability. The estimated basecalling error probability may be translated to Phred-style quality scores by the following equation: QV = -10 x log (Probability of Error). The quality value model 1010 may be a convolutional neural network. The quality value model 1010 may have several hidden layers with a logistic regression layer. The hypothesis functions, such as sigmoid function, may be utilized in the logistic regression layer to predict the estimated error probability based on the input scan probabilities. The quality value model 1010 may comprise one or more trained models that may be selected to be utilized. The selection may be based on minimum evaluation loss or error rate. The quality value model 1010 may be trained in accordance with the process depicted in Figure 12. The estimated basecalling error probabilities are then associated with the basecalls for the assembled scan windows.

The sequencer 1012 receives the basecalls for the input trace sequence, the assembled scan windows, and the estimated basecalling error probabilities. The sequencer 1012 then finds the scan positions for the basecalls based on the output label probabilities from CTC networks and basecalls from decoders. The sequencer 1012 may utilize a deque max finder algorithm. The sequencer 1012 thus generates the output basecall sequence and estimated error probability.

In some embodiments, data augmentation techniques such as adding noise, spikes, dye blobs or other data artefacts or simulated sequencing trace may be utilized. These techniques may improve the robustness of the basecaller system 1000. Generative Adversarial Nets (GANs) may be utilized to implement these techniques.

Referring to Figure 11, a scan label model training method 1100 receives datasets (block 1102). The datasets may include pure base datasets and mixed base datasets. For example, the pure base dataset may comprise ~49M basecalls and the mixed base dataset may comprise ~13.4M basecalls. The mixed base data set may be composed primarily of pure bases with occasional mixed bases. For each sample in the dataset, the entire trace is divided into scan windows (block 1104). Each scan window may have 500 scans. The trace may be a sequence of preprocessed dye RFUs. Additionally, the scan windows for each sample can be shifted by 250 scans to minimize the bias of the scan position on training. The annotated basecalls are then determined for each scan window (block 1106). These are utilized as the target sequence during the training. The training samples are then constructed (block 1108). Each of them may comprise the scan window with 500 scans and the respective annotated basecalls. A BRNN with one or more layers of LSTM is initialized (block 1110). The BRNN may utilize other units similar to the LSTM, such as a Gated Recurrent Unit (GRU). A Softmax layer may be utilized as the output layer of the LSTM BRNN, which outputs the label probabilities for all scans in the input scan window.

The training samples are then applied to the BRNN (block 1112). The label probabilities for all scans in the input scan windows are output (block 1114). The loss between the output scan label probabilities and the target annotated basecalls are calculated. A Connectionist Temporal Classification (CTC) loss function may be utilized to calculate the loss between the output scan label probabilities and the target annotated basecalls. A mini-batch of training samples is then selected (block 1118). The mini-batch may be selected randomly from the training dataset at each training step. The weights of the networks are updated to minimize the CTC loss against the mini-batch of training samples (block 1120). An Adam optimizer or other gradient descent optimizer may be utilized to update the weights. The networks are then saved as a model (block 1122). In some embodiments, the model is saved during specific training steps. The scan label model training method 1100 then determines whether a predetermined number of training steps has been reached (decision block 1124). If not, the scan label model training method 1100 is re-performed from block 1112 utilizing the network with the updated weights (i.e., the next iteration of the network). Once the predetermined number of training steps are performed, the saved models are evaluated (block 1126). The evaluation may be performed utilizing an independent subset of samples in the validation dataset, which are not included in the training process. The best trained models are then selected based on minimum evaluation loss or error rate from the trained models. These model(s) may then be utilized by the basecaller system 1000.

In some embodiments, data augmentation techniques such as adding noise, spikes, dye blobs or other data artefacts or simulated sequencing trace by Generative Adversarial Nets (GANs) may be utilized to improve the robustness of the models. Also, during training, other techniques, such as drop-out or weight decay, may be used to improve the generality of the models.

Referring to Figure 12, a QV model training method 1200 utilize a trained network and decoder to calculate scan label probabilities, basecalls, and their scan positions (block 1202). The trained network and decoder may be those depicted in Figure 10. Training samples are constructed for QV training (block 1204). The scan probabilities around the center scan position for each basecall may be utilized and all basecalls may be assigned into two categories: correct basecalls or incorrect basecalls. A convolution neural network (CNN) with several hidden layers with a logistic regression layer may be utilized to be trained (block 1206). The CNN and logistic regression layer may be initialized. An estimated error probability may be predicted based on the input scan probabilities (block 1208). A hypothesis function, such as a sigmoid function, may be utilized in the logistic regression layer to predict the estimated error probability based on the input scan probabilities. A loss between the predicted error probabilities and the basecall categories is then calculated (block 1210). The cost functions for logistic regression such as logistic loss (or called as cross-entropy loss) may be used to calculate the loss between the predicted error probabilities and the basecall categories.

A mini-batch of training samples is then selected (block 1212). The mini-batch may be selected randomly from the training dataset at each training step. The weights of the networks are updated to minimize the logistic loss against the mini-batch of training samples (block 1214). An Adam optimizer or other gradient descent optimizer may be utilized to update the weights. The networks are then saved as a model (block 1216). In some embodiments, the model is saved during specific training steps. The QV model training method 1200 then determines whether a predetermined number of training steps has been reached (decision block 1218). If not, the QV model training method 1200 is re-performed from block 1206 utilizing the network with the updated weights (i.e., the next iteration of the network). Once the predetermined number of training steps are performed, the saved models are evaluated (block 1220). The models may be evaluated by an independent subset of samples in the validation dataset, which are not included in the training process. The selected trained models may be those with minimum evaluation loss or error rate.

Figure 13 is an example block diagram of a computing device 1300 that may incorporate embodiments of the present invention. Figure 13 is merely illustrative of a machine system to carry out aspects of the technical processes described herein, and does not limit the scope of the claims. One of ordinary skill in the art would recognize other variations, modifications, and alternatives. In one embodiment, the computing device 1300 typically includes a monitor or graphical user interface 1302, a data processing system 1320, a communication network interface 1312, input device(s) 1308, output device(s) 1306, and the like.

As depicted in Figure 13, the data processing system 1320 may include one or more processor(s) 1304 that communicate with a number of peripheral devices via a bus subsystem 1318. These peripheral devices may include input device(s) 1308, output device(s) 1306, communication network interface 1312, and a storage subsystem, such as a volatile memory 1310 and a non-volatile memory 1314.

The volatile memory 1310 and/or the nonvolatile memory 1314 may store computer-executable instructions and thus forming logic 1322 that when applied to and executed by the processor(s) 1304 implement embodiments of the processes disclosed herein.

The input device(s) 1308 include devices and mechanisms for inputting information to the data processing system 1320. These may include a keyboard, a keypad, a touch screen incorporated into the monitor or graphical user interface 1302, audio input devices such as voice recognition systems, microphones, and other types of input devices. In various embodiments, the input device(s) 1308 may be embodied as a computer mouse, a trackball, a track pad, a joystick, wireless remote, drawing tablet, voice command system, eye tracking system, and the like. The input device(s) 1308 typically allow a user to select objects, icons, control areas, text and the like that appear on the monitor or graphical user interface 1302 via a command such as a click of a button or the like.

The output device(s) 1306 include devices and mechanisms for outputting information from the data processing system 1320. These may include the monitor or graphical user interface 1302, speakers, printers, infrared LEDs, and so on as well understood in the art.

The communication network interface 1312 provides an interface to communication networks (e.g., communication network 1316) and devices external to the data processing system 1320. The communication network interface 1312 may serve as an interface for receiving data from and transmitting data to other systems. Embodiments of the communication network interface 1312 may include an Ethernet interface, a modem (telephone, satellite, cable, ISDN), (asynchronous) digital subscriber line (DSL), FireWire, USB, a wireless communication interface such as Bluetooth or WiFi, a near field communication wireless interface, a cellular interface, and the like.

The communication network interface 1312 may be coupled to the communication network 1316 via an antenna, a cable, or the like. In some embodiments, the communication network interface 1312 may be physically integrated on a circuit board of the data processing system 1320, or in some cases may be implemented in software or firmware, such as "soft modems", or the like.

The computing device 1300 may include logic that enables communications over a network using protocols such as HTTP, TCP/IP, RTP/RTSP, IPX, UDP and the like.

The volatile memory 1310 and the nonvolatile memory 1314 are examples of tangible media configured to store computer readable data and instructions forming logic to implement aspects of the processes described herein. Other types of tangible media include removable memory (e.g., pluggable USB memory devices, mobile device SIM cards), optical storage media such as CD-ROMS, DVDs, semiconductor memories such as flash memories, non-transitory read-only-memories (ROMS), battery-backed volatile memories, networked storage devices, and the like. The volatile memory 1310 and the nonvolatile memory 1314 may be configured to store the basic programming and data constructs that provide the functionality of the disclosed processes and other embodiments thereof that fall within the scope of the present invention.

Logic 1322 that implements embodiments of the present invention may be formed by the volatile memory 1310 and/or the nonvolatile memory 1314 storing computer readable instructions. Said instructions may be read from the volatile memory 1310 and/or nonvolatile memory 1314 and executed by the processor(s) 1304. The volatile memory 1310 and the nonvolatile memory 1314 may also provide a repository for storing data used by the logic 1322.

The volatile memory 1310 and the nonvolatile memory 1314 may include a number of memories including a main random access memory (RAM) for storage of instructions and data during program execution and a read only memory (ROM) in which read-only non-transitory instructions are stored. The volatile memory 1310 and the nonvolatile memory 1314 may include a file storage subsystem providing persistent (non-volatile) storage for program and data files. The volatile memory 1310 and the nonvolatile memory 1314 may include removable storage systems, such as removable flash memory.

The bus subsystem 1318 provides a mechanism for enabling the various components and subsystems of data processing system 1320 communicate with each other as intended. Although the communication network interface 1312 is depicted schematically as a single bus, some embodiments of the bus subsystem 1318 may utilize multiple distinct busses.

It will be readily apparent to one of ordinary skill in the art that the computing device 1300 may be a device such as a smartphone, a desktop computer, a laptop computer, a rack-mounted computer system, a computer server, or a tablet computer device. As commonly known in the art, the computing device 1300 may be implemented as a collection of multiple networked computing devices. Further, the computing device 1300 will typically include operating system logic (not illustrated) the types and nature of which are well known in the art.

## Exemplary Embodiments

A new deep learning-based basecaller, Deep Basecaller, was developed to improve mixed basecalling accuracy and pure basecalling accuracy especially at 5' and 3' ends, and to increase read length for Sanger sequencing data in capillary electrophoresis instruments.

Bidirectional Recurrent Neural Networks (BRNNs) with Long Short-Term Memory (LSTM) units have been successfully engineered to basecall Sanger sequencing data by translating the input sequence of dye RFUs (relative fluoresce units) collected from CE instruments to the output sequence of basecalls. Large annotated Sanger Sequencing datasets, which include ~49M basecalls for the pure base data set and ~13.4M basecalls for the mixed base data set, were used to train and test the new deep learning based basecaller.

Below is an exemplary workflow of algorithms used for Deep Basecaller:

1. For each sample in the training pure or mixed base dataset, divide the entire analyzed trace, the sequence of preprocessed dye RFUs (relative fluoresce units), into scan windows with length 500 scans. The scan windows for each sample can be shifted by 250 scans to minimize the bias of the scan position on training.

2. Determine the annotated basecalls for each scan window- as the target sequence during the training.

3. Construct training samples, each of them consisting of the scan window with 500 scans and the respective annotated basecalls.

4. Use Bidirectional Recurrent Neural Network (BRNN) with one or more layers of LSTM or similar units such as GRU (Gated Recurrent Unit) as the network to be trained.

5. Use Softmax layer as the output layer of LSTM BRNN, which outputs the label probabilities for all scans in the input scan window.

6. Apply a Connectionist Temporal Classification (CTC) loss function to calculate the loss between the output scan label probabilities and the target annotated basecalls.

7. Use a gradient descent optimizer to update the weights of the networks described above to minimize the CTC loss against a minibatch of training samples, which are randomly selected from the training dataset at each training step.

8. Continue the training process until the prefixed number of training steps is reached and save the trained networks for specified training steps.

9. Evaluate the trained models, which are saved during the training process, by an independent subset of samples in the validation dataset, which are not included in the training process. Select the trained models with minimum evaluation loss or error rate as the best trained models.

10. For a sequencing sample, divide the entire trace into scan windows with 500 scans shifted by 250 scans. Apply the selected trained models on those scan windows to output the scan label probabilities for all scan windows.

11. Assemble the label probabilities for all scan windows together to construct the label probabilities for the entire trace of the sequencing sample.

12. Use prefix Beam search or other decoders on the assembled label probabilities to find the basecalls for the sequencing sample

13. Use dequeue max finder algorithm to find the scan positions for all basecalls based on the output label probabilities from CTC networks and basecalls from decoders.

14. Deep learning models described above can be applied on raw traces (the sequence of raw dye RFUs) or raw spectrum data collected in the CE instruments directly, prior to processing by a basecaller (such as KB Basecaller).

15. Data augmentation techniques such as adding noise, spikes, dye blobs or other data artefacts or simulated sequencing trace by Generative Adversarial Nets (GANs) can be used to improve the robustness of the trained Deep Basecaller.

16. During the training, the techniques such as dropout or weight decay can be used to improve the generality of the trained deep basecaller.

Below are exemplary details about quality value (QV) algorithms for Deep Basecaller:

1. Apply the trained CTC network and decoder on all samples in the training set to obtain/calculate scan label probabilities, basecalls and their scan positions.

2. Construct training samples for QV training by using the scan probabilities around the center scan position for each basecall and assign all basecalls into two categories: correct basecalls or incorrect basecalls.

3. Use convolutional neural network with several hidden layers with a logistic regression layer as the network to be trained.

4. The hypothesis functions such as sigmoid function can be used in the logistic regression layer to predict the estimated error probability based on the input scan probabilities. The cost functions for logistic regression such as logistic loss (or called as cross-entropy loss) can be used to calculate the loss between the predicted error probabilities and the basecall categories.

5. Use an Adam optimizer or other gradient descent optimizers to update the weights of the networks described above to minimize the logistic loss against a minibatch of training samples, which are randomly selected from the training dataset at each training step.

6. Continue the training process until the prefixed number of training steps is reached and save the trained networks for specified training steps.

7. Evaluate the trained models, which are saved during training process, by an independent subset of samples in the validation dataset, which are not included in the training process. Select the trained models with minimum evaluation loss or error rate as the best trained models.

8. The trained QV model will take the scan probabilities around basecall positions as the input and then output the estimated basecalling error probability, which can be translated to Phred-style quality scores by the following equation:

$$QV = -10 \times \log(\text{Probability of Error}).$$

Deep Basecaller may use deep learning approaches described above to generate the scan probabilities, basecalls with their scan positions and quality values.

## Alternative Embodiments

LSTM BRNN or similar networks such as GRU BRNN with sequence-to-sequence architecture such as the encoder-decoder model with or without attention mechanism may also be used for basecalling Sanger sequencing data.

Segmental recurrent neural networks (SRNNs) can be also used for Deep Basecaller. In this approach, bidirectional recurrent neural nets are used to compute the "segment embeddings" for the contiguous subsequences of the input trace or input trace segments, which can be used to define compatibility scores with the output basecalls. The compatibility scores are then integrated to output a joint probability distribution over segmentations of the input and basecalls of the segments.

The frequency data of overlapped scan segments similar to Mel-frequency cepstral coefficients (MFCCs) in speech recognition can be used as the input for Deep Basecaller. Simple convolutional neural networks or other simple networks can be used on the overlapped scan segments to learn local features, which are then used as the input for LSTM BRNN or similar networks to train Deep Basecaller.

When the scans and basecalls are aligned or the scan boundaries for basecalls are known for the training data set, loss functions other than CTC loss such as Softmax cross entropy loss functions can be used with LSTM BRNN or similar networks, and such networks can be trained to classify the scans into basecalls. Alternatively, convolutional neural networks such as R-CNN (Region-based Convolutional Neural Networks) can be trained to segment the scans and then basecall each scan segment.

## Implementation and Additional Terminology

Terms used herein should be accorded their ordinary meaning in the relevant arts, or the meaning indicated by their use in context, but if an express definition is provided, that meaning controls.

"Circuitry" in this context refers to electrical circuitry having at least one discrete electrical circuit, electrical circuitry having at least one integrated circuit, electrical circuitry having at least one application specific integrated circuit, circuitry forming a general purpose computing device configured by a computer program (e.g., a general purpose computer configured by a computer program which at least partially carries out processes or devices described herein, or a microprocessor configured by a computer program which at least partially carries out processes or devices described herein), circuitry forming a memory device (e.g., forms of random access memory), or circuitry forming a communications device (e.g., a modem, communications switch, or optical-electrical equipment).

"Firmware" in this context refers to software logic embodied as processor-executable instructions stored in read-only memories or media.

"Hardware" in this context refers to logic embodied as analog or digital circuitry.

"Logic" in this context refers to machine memory circuits, non transitory machine readable media, and/or circuitry which by way of its material and/or material-energy configuration comprises control and/or procedural signals, and/or settings and values (such as resistance, impedance, capacitance, inductance, current/voltage ratings, etc.), that may be applied to influence the operation of a device. Magnetic media, electronic circuits, electrical and optical memory (both volatile and nonvolatile), and firmware are examples of logic. Logic specifically excludes pure signals or software per se (however does not exclude machine memories comprising software and thereby forming configurations of matter).

"Software" in this context refers to logic implemented as processor-executable instructions in a machine memory (e.g. read/write volatile or nonvolatile memory or media).

Herein, references to "one embodiment" or "an embodiment" do not necessarily refer to the same embodiment, although they may. Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively, unless expressly limited to a single one or multiple ones. Additionally, the words "herein," "above," "below" and words of similar import, when used in this application, refer to this application as a whole and not to any particular portions of this application. When the claims use the word "or" in reference to a list of two or more items, that word covers all of the following interpretations of the word: any of the items in the list, all of the items in the list and any combination of the items in the list, unless expressly limited to one or the other. Any terms not expressly defined herein have their conventional meaning as commonly understood by those having skill in the relevant art(s).

Various logic functional operations described herein may be implemented in logic that is referred to using a noun or noun phrase reflecting said operation or function. For example, an association operation may be carried out by an "associator" or "correlator". Likewise, switching may be carried out by a "switch", selection by a "selector", and so on.

## Claims

**1.** A neural network control system comprising:

a trace generator coupled to a Sanger Sequencer and generating a trace for a biological sample;
a segmenter to divide the trace into scan windows;
an aligner to shift the scan windows;
logic to determine associated annotated basecalls for each of the scan windows to generate target annotated basecalls for use in training;
a bi-directional recurrent neural network, BRNN, comprising:

at least one long short term memory, LSTM, or general recurrent unit, GRU, layer;
an output layer configured to output scan label probabilities for all scans in a scan window;
a Connectionist Temporal Classification loss function to calculate the loss between the output scan label probabilities and the target annotated basecalls; and
a gradient descent optimizer configured as a closed loop feedback control to the BRNN to update weights of the BRNN to minimize the loss against a minibatch of training samples randomly selected from the target annotated basecalls at each training step.

**2.** The system of claim 1, further comprising: each of the scan windows comprising 500 scans shifted by 250 scans.

**3.** The system of claim 1, further comprising:
an aggregator to assemble the label probabilities for all scan windows to generate label probabilities for the entire trace, preferably further comprising:
a dequeue max finder algorithm to identify scan positions for the basecalls based on an output of the Connectionist Temporal Classification loss function and the basecalls, or a prefix beam search decoder to transform the label probabilities for the entire trace into basecalls for the biological sample, preferably, wherein the basecalls are at 5' and 3' ends of the biological sample.

**4.** The system of claim 1, wherein the trace is a sequence of raw dye relative fluorescence units.

**5.** The system of claim 1, wherein the trace is raw spectrum data collected from one or more capillary electrophoresis genetic analyzer.

**6.** The system of claim 1, further comprising: at least one generative adversarial network configured to inject noise in the trace, at least one generative adversarial network configured to inject spikes into the trace, or at least one generative adversarial network configured to inject dye blob artifacts into the trace.

**7.** A process control method, comprising:

operating a Sanger Sequencer to generate a trace for a biological sample;
dividing the trace into scan windows; shifting the scan windows;
determining associated annotated basecalls for each of the scan windows to generate target annotated basecalls;
inputting the scan windows to a bi-directional recurrent neural network, BRNN, comprising: at least one long short term memory, LSTM, or general recurrent unit, GRU, layer; an output layer configured to output scan label probabilities for all scans in a scan window; a Connectionist Temporal Classification loss function to calculate the loss between the output scan label probabilities and the target annotated basecalls; and
applying the loss through a gradient descent optimizer configured as a closed loop feedback control to the BRNN to update weights of the BRNN to minimize the loss against a minibatch of training samples randomly selected from the target annotated basecalls at each training step.

**8.** The method of claim 7, further comprising:
each of the scan windows comprising 500 scans shifted by 250 scans.

**9.** The method of claim 7, further comprising:
assembling the label probabilities for all scan windows to generate label probabilities for the entire trace.

**10.** The method of claim 9. further comprising: identifying scan positions for the basecalls based on an output of the Connectionist Temporal Classification loss function and the basecalls.

**11.** The method of claim 9, further comprising: decoding the label probabilities for the entire trace into basecalls for the biological sample, preferably, wherein the basecalls are at 5' and 3' ends of the biological sample.

**12.** The method of claim 7, wherein the trace is one of a sequence of raw dye relative fluorescence units, or raw spectrum data collected from one or more capillary electrophoresis genetic analyzer.

**13.** The method of claim 7, further comprising: at least one generative adversarial network configured to inject one or more of noise, spikes, or dye blog artifacts into the trace.

**Patentansprüche**

**1.** Neurales Netzwerk-Steuerungssystem, umfassend:

einen Spurengenerator, der mit einem Sanger-Sequenzer gekoppelt ist und eine Spur für eine biologische Probe erzeugt;
einen Segmentierer zum Aufteilen der Spur in Scanfenster;
einen Aligner zum Verschieben der Scanfenster;
Logik zum Bestimmen zugehöriger annotierter Basecalls für jedes der Scan-Fenster, um zielannotierte Basecalls zur Verwendung beim Training zu generieren;
ein bidirektionales rekurrentes neurales Netzwerk, BRNN, bestehend aus:

mindestens eine Long Short Term Memory-Schicht (LSTM) oder General Recurrent Unit-Schicht (GRU);
eine Ausgabeebene, die konfiguriert ist, um Scanlabel-Wahrscheinlichkeiten für alle Scans in einem Scan-Fenster auszugeben;
eine konnektionistische temporale Klassifizierungsverlustfunktion zum Berechnen des Verlusts zwischen den ausgegebenen Scanlabel-Wahrscheinlichkeiten und den zielannotierten Basecalls; und
einen Gradientenabstiegsoptimierer, der als eine geschlossene Rückkopplungsschleife für das BRNN konfiguriert ist, um die Gewichte des BRNN zu aktualisieren und so den Verlust gegenüber einem Minibatch von Trainingsbeispielen, die bei jedem Trainingsschritt zufällig aus den zielannotierten Basecalls ausgewählt werden, zu minimieren.

**2.** System nach Anspruch 1, ferner umfassend: Jjedes der Scanfenster umfasst 500 Scans, die um 250 Scans verschoben sind.

**3.** System nach Anspruch 1, ferner umfassend:
einen Aggregator zum Zusammenstellen der Label-Wahrscheinlichkeiten für alle Scan-Fenster, um Label-Wahrscheinlichkeiten für die gesamte Spur zu erzeugen, vorzugsweise ferner umfassend:
ein Dequeue-Max-Finder-Algorithmus zum Identifizieren von ScanPositionen für die Basecalls basierend auf einer Ausgabe der konnektionistischen temporalen Klassifizierungsverlustfunktion und den Basecalls, oder ein Prefix-Beam-Search-Decoder zum Umwandeln der Label-Wahrscheinlichkeiten für die gesamte Spur in Basecalls für die biologische Probe, wobei sich vorzugsweise die Basecalls an den 5'- und 3'-Enden der biologischen Probe befinden.

**4.** System nach Anspruch 1, wobei die Spur eine Sequenz von Rohfarbstoffbezogenen Fluoreszenzeinheiten ist.

**5.** System nach Anspruch 1, wobei die Spur aus Rohspektrumdaten besteht, die von einem oder mehreren genetischen Kapillarelektrophorese-Analysatoren gesammelt wurden.

**6.** System nach Anspruch 1, ferner umfassend: mindestens ein generatives kontradiktorisches Netzwerk, das zum Einfügen von Rauschen in die Spur konfiguriert ist, mindestens ein generatives kontradiktorisches Netzwerk, das zum Einfügen von Peaks in die Spur konfiguriert ist, oder mindestens ein generatives kontradiktorisches Netzwerk, das zum Einfügen von Dye-Blob-Artefakten in die Spur konfiguriert ist.

**7.** Verfahren zur Prozesssteuerung, umfassend:

Bedienen eines Sanger-Sequenzers zum Erzeugen einer Spur für eine biologische Probe;
Aufteilen der Spur in Scanfenster; Verschieben der Scanfenster;
Bestimmen zugehöriger annotierter Basecalls für jedes der Scan-Fenster, um annotierte Ziel-Basecalls zu erzeugen;
Eingeben der Scanfenster in ein bidirektionales rekurrentes neurales Netzwerk (BRNN), umfassend: mindestens eine Schicht mit Long Short Term Memory(LSTM) oder General Recurrent Unit (GRU); eine Ausgabeebene, die konfiguriert ist, um Scanlabel-Wahrscheinlichkeiten für alle Scans in einem Scan-Fenster auszugeben;

eine konnektionistische temporale Klassifizierungsverlustfunktion zum Berechnen des Verlusts zwischen den ausgegebenen Scanlabel-Wahrscheinlichkeiten und den zielannotierten Basecalls; und

Anwenden des Verlusts durch einen Gradientenabstiegsoptimierer, der als geschlossene Rückkopplungsschleife für das BRNN konfiguriert ist, um die Gewichte des BRNN zu aktualisieren und so den Verlust gegenüber einem Minibatch von Trainingsbeispielen, die bei jedem Trainingsschritt zufällig aus den mit Zielannotationen versehenen Basecalls ausgewählt werden, zu minimieren.

**8.** Verfahren nach Anspruch 7, ferner umfassend: jedes der Scanfenster umfasst 500 Scans, die um 250 Scans verschoben sind.

**9.** Verfahren nach Anspruch 7, ferner umfassend: Zusammenstellen der Label-Wahrscheinlichkeiten für alle Scanfenster, um Label-Wahrscheinlichkeiten für die gesamte Spur zu generieren.

**10.** Verfahren nach Anspruch 9, ferner umfassend: Identifizieren von Scanpositionen für die Basecalls basierend auf einer Ausgabe der konnektionistischen temporalen Klassifizierungsverlustfunktion und den Basecalls.

**11.** Verfahren nach Anspruch 9, ferner umfassend: Dekodieren der Markierungswahrscheinlichkeiten für die gesamte Spur in Basecalls für die biologische Probe, wobei sich die Basecalls vorzugsweise an den 5'- und 3'-Enden der biologischen Probe befinden.

**12.** Verfahren nach Anspruch 7, wobei die Spur eine Sequenz von Rohfarbstoff-Fluoreszenzeinheiten oder Rohspektrumdaten ist, die von einem oder mehreren genetischen Kapillarelektrophorese-Analysegeräten gesammelt wurden.

**13.** Verfahren nach Anspruch 7, ferner umfassend: mindestens ein generatives kontradiktorisches Netzwerk, das konfiguriert ist, um eines oder mehrere von Rauschen, Spitzen oder Dye-Blog-Artefakten in die Spur einzufügen.

## Revendications

**1.** Système de commande à réseau neuronal, comprenant :

un générateur de traces couplé à un séquenceur Sanger et générant une trace pour un échantillon biologique ;

un segmenteur pour diviser la trace en fenêtres de balayage ;

un aligneur pour décaler les fenêtres de balayage ;

une logique permettant de déterminer des appels de base annotés associés pour chacune des fenêtres de balayage afin de générer des appels de base annotés cibles pour une utilisation dans l'entraînement ;

un réseau neuronal récurrent bidirectionnel, BRNN, comprenant :

au moins une couche de mémoire à long terme et à court terme, LSTM, ou une couche d'unité récurrente générale, GRU ;

une couche de sortie configurée pour délivrer en sortie des probabilités d'étiquetage de balayage pour l'ensemble des balayages dans une fenêtre de balayage ;

une fonction de perte de classification temporelle de connexion pour calculer la perte entre les probabilités d'étiquetage de balayage de sortie et les appels de base annotés cibles ; et

un optimiseur de descente de gradient configuré comme une commande en boucle fermée à rétroaction du BRNN pour mettre à jour les poids du BRNN afin de minimiser la perte par rapport à un mini-ensemble d'échantillons d'entraînement sélectionnés de manière aléatoire parmi les appels de base annotés cibles à chaque étape de l'entraînement.

**2.** Système selon la revendication 1, comprenant en outre : chacune des fenêtres de balayage comprenant 500 balayages décalés de 250 balayages.

**3.** Système selon la revendication 1, comprenant en outre :

un agrégateur pour assembler les probabilités d'étiquetage pour toutes les fenêtres de balayage afin de générer des probabilités d'étiquetage pour l'ensemble de la trace, comprenant de préférence en outre :

un algorithme de recherche de maximum avec file double pour identifier des positions de balayage pour les appels de base sur la base d'une sortie de la fonction de perte de classification temporelle de connexion et des appels de base, ou un décodeur de recherche de faisceau de préfixe pour transformer les probabilités d'étiquetage pour la trace entière en appels de base pour l'échantillon biologique, de préférence, dans lequel les appels de base sont aux extrémités 5' et 3' de l'échantillon biologique.

**4.** Système selon la revendication 1, dans lequel la trace est une séquence d'unités relatives brutes de fluorescence de colorant.

**5.** Système selon la revendication 1, dans lequel la trace est constituée de données spectrales brutes recueillies par un ou plusieurs analyseurs génétiques par électrophorèse capillaire.

**6.** Système selon la revendication 1, comprenant en outre : au moins un réseau antagoniste génératif configuré pour injecter du bruit dans la trace, au moins un réseau antagoniste génératif étant configuré pour injecter des pics dans la trace, ou au moins un réseau antagoniste génératif configuré pour injecter des artéfacts de taches de colorant dans la trace.

**7.** Procédé de commande de processus, comprenant :

l'utilisation d'un séquenceur Sanger pour générer une trace pour un échantillon biologique ;
la division de la trace en fenêtres de balayage ; le décalage des fenêtres d'analyse ;
la détermination d'appels de base annotés associés pour chacune des fenêtres de balayage afin de générer des appels de base annotés cibles ;
l'entrée des fenêtres de balayage dans un réseau neuronal récurrent bidirectionnel, BRNN, comprenant : au moins une couche de mémoire à long terme, LSTM, ou une couche d'unité récurrente générale, GRU ; une couche de sortie configurée pour délivrer en sortie des probabilités d'étiquetage de balayage pour l'ensemble des balayages dans une fenêtre de balayage ; une fonction de perte de classification temporelle de connexion pour calculer la perte entre les probabilités d'étiquetage de balayage de sortie et les appels de base annotés cibles ; et
l'application de la perte par le biais d'un optimiseur de descente de gradient configuré comme une commande en boucle fermée à rétroaction du BRNN pour mettre à jour des poids du BRNN afin de minimiser la perte par rapport à un mini-ensemble d'échantillons d'entraînement sélectionnés de manière aléatoire parmi les appels de base annotés cibles à chaque étape d'entraînement.

**8.** Procédé selon la revendication 7, comprenant en outre :
chacune des fenêtres de balayage comprenant 500 balayages décalés de 250 balayages.

**9.** Procédé selon la revendication 7, comprenant en outre :
l'assemblage des probabilités d'étiquetage pour toutes les fenêtres de balayage afin de générer des probabilités d'étiquetage pour l'ensemble de la trace.

**10.** Procédé selon la revendication 9, comprenant en outre :
l'identification de positions de balayage pour les appels de base sur la base d'une sortie de la fonction de perte de la classification temporelle de connexion et des appels de base.

**11.** Procédé selon la revendication 9, comprenant en outre :
le décodage des probabilités d'étiquetage pour l'ensemble de la trace en appels de base pour l'échantillon biologique, de préférence, dans lequel les appels de base sont aux extrémités 5' et 3' de l'échantillon biologique.

**12.** Procédé selon la revendication 7, dans lequel la trace est l'une parmi une séquence d'unités relatives brutes de fluorescence de colorant, ou des données spectrales brutes recueillies à partir d'un ou plusieurs analyseurs génétiques par électrophorèse capillaire.

**13.** Procédé selon la revendication 7, comprenant en outre : au moins un réseau antagoniste génératif configuré pour injecter un ou plusieurs parmi bruit, pics ou artéfacts de taches de colorant dans la trace.

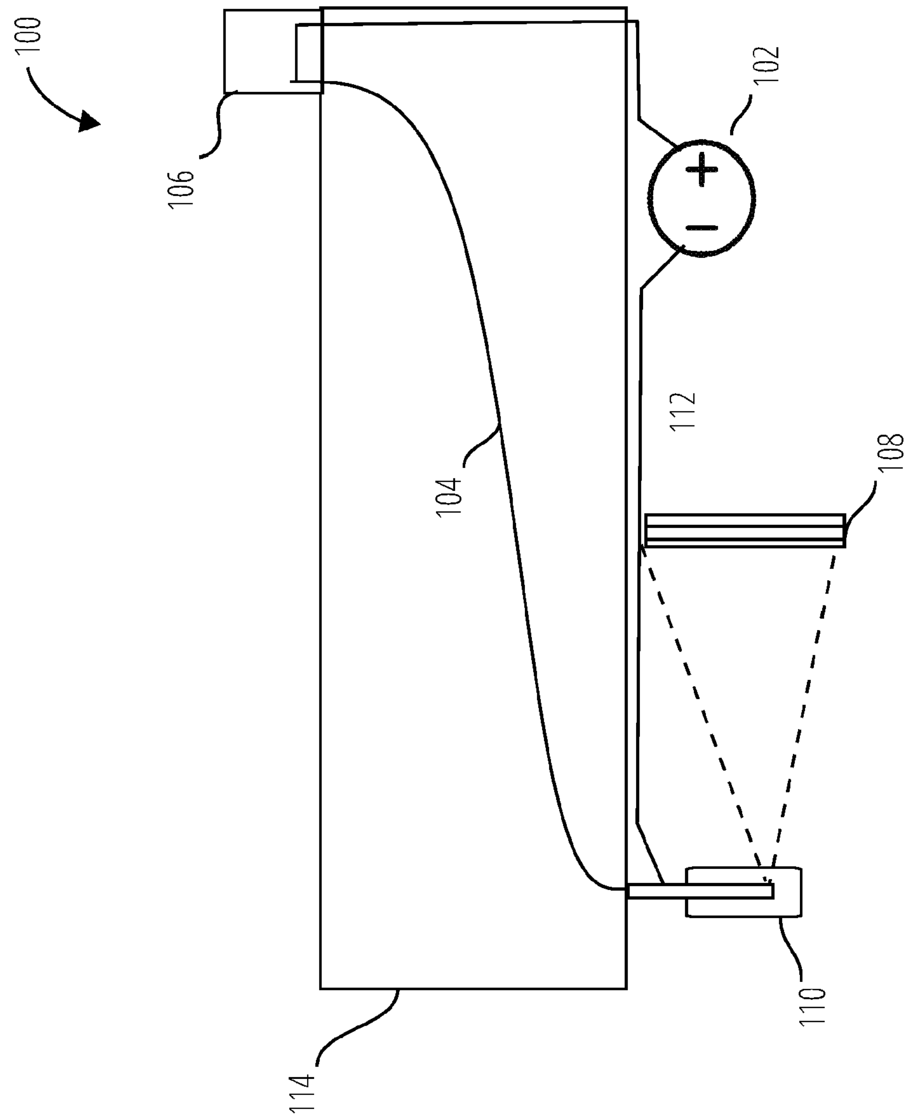


(PRIOR ART)

FIG. 1

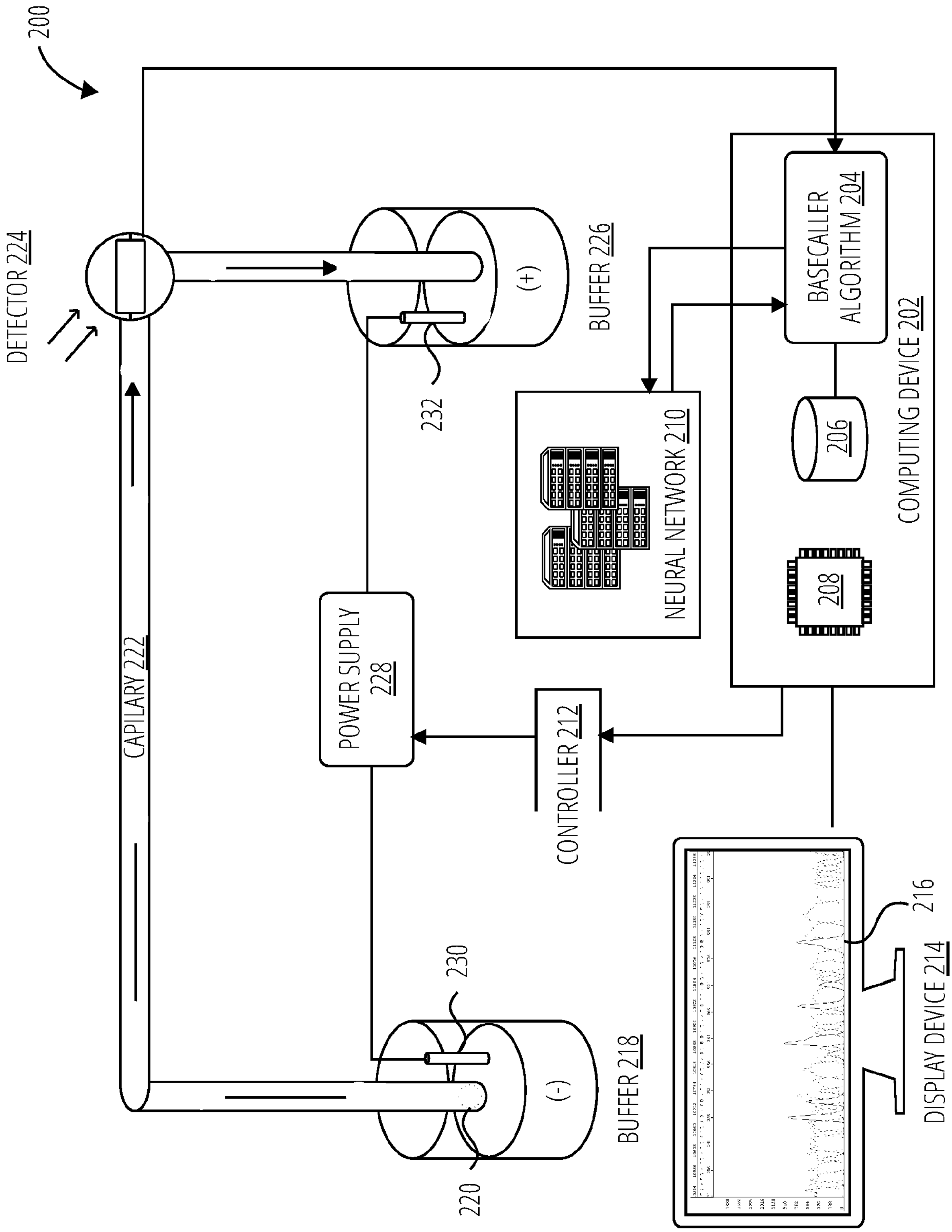
200
DETECTOR 224
BUFFER 226
(+)
232
CAPILARY 222
POWER SUPPLY 228
CONTROLLER 212
BUFFER 218
(-)
230
220
NEURAL NETWORK 210
BASECALLER ALGORITHM 204
206
208
COMPUTING DEVICE 202
DISPLAY DEVICE 214
216

FIG. 2

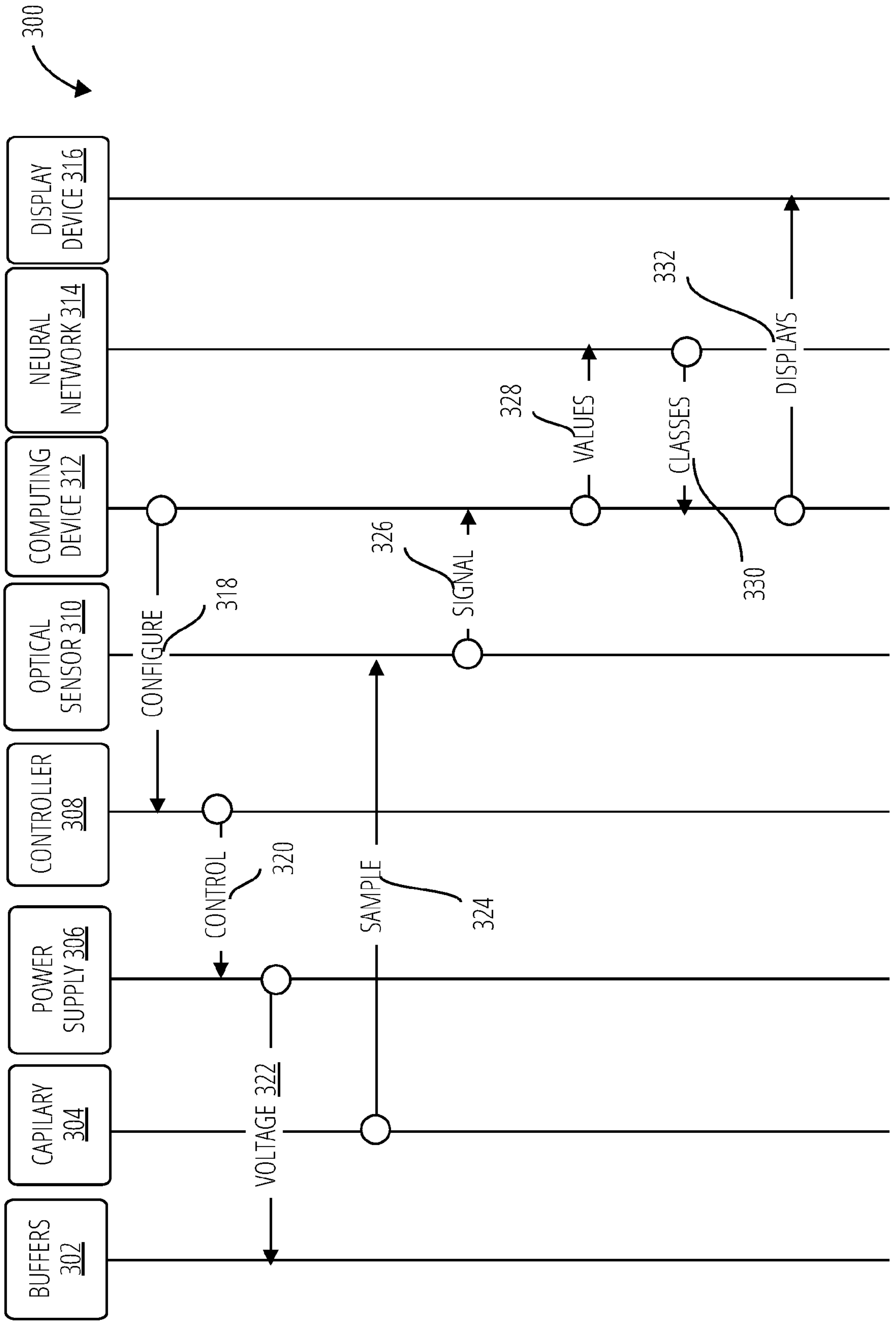
300
BUFFERS 302
CAPILARY 304
POWER SUPPLY 306
CONTROLLER 308
OPTICAL SENSOR 310
COMPUTING DEVICE 312
NEURAL NETWORK 314
DISPLAY DEVICE 316
CONFIGURE
318
CONTROL
320
VOLTAGE 322
SAMPLE
324
SIGNAL
326
VALUES
328
CLASSES
330
DISPLAYS
332

FIG. 3

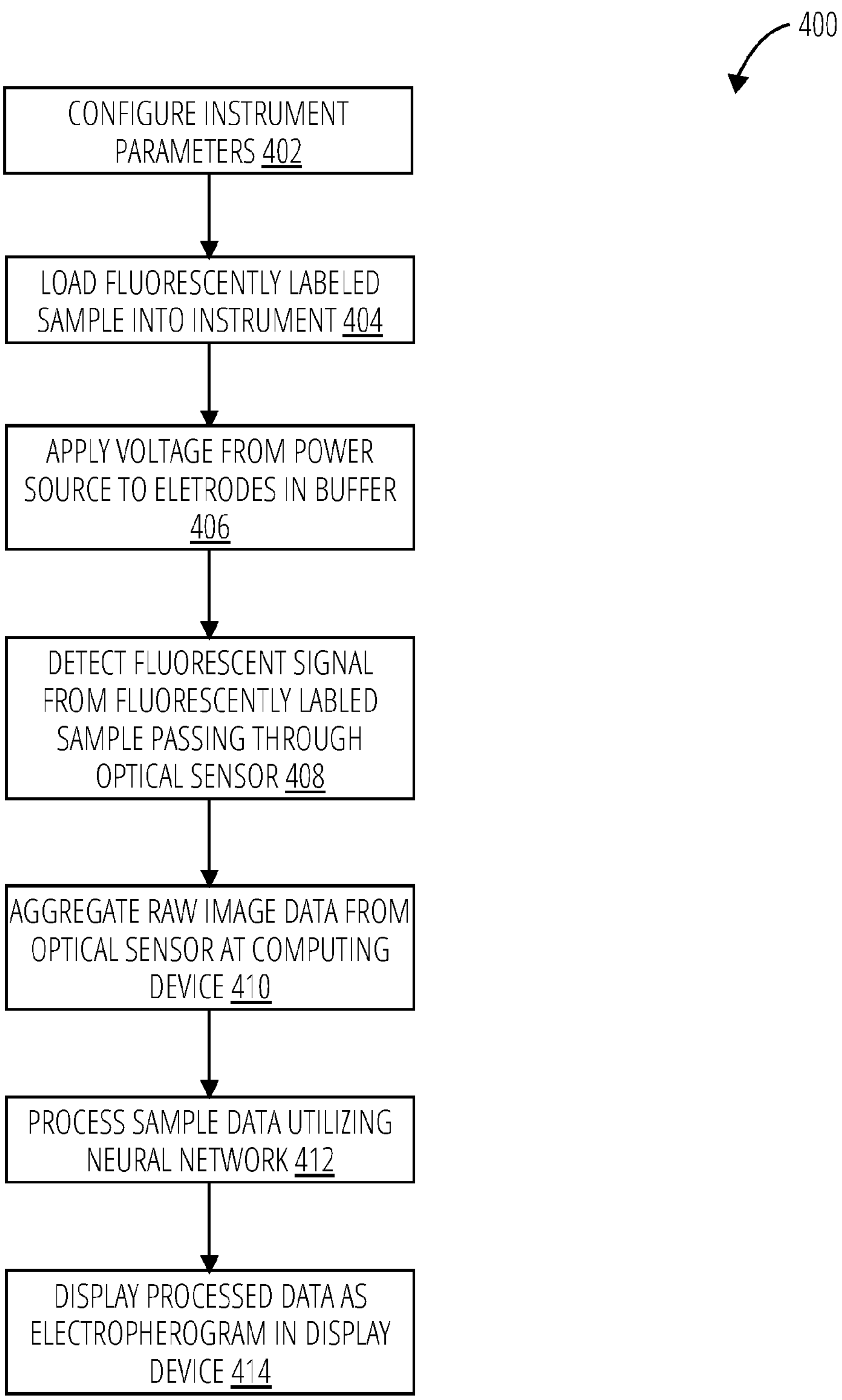
400
CONFIGURE INSTRUMENT PARAMETERS 402
LOAD FLUORESCENTLY LABELED SAMPLE INTO INSTRUMENT 404
APPLY VOLTAGE FROM POWER SOURCE TO ELETRODES IN BUFFER 406
DETECT FLUORESCENT SIGNAL FROM FLUORESCENTLY LABLED SAMPLE PASSING THROUGH OPTICAL SENSOR 408
AGGREGATE RAW IMAGE DATA FROM OPTICAL SENSOR AT COMPUTING DEVICE 410
PROCESS SAMPLE DATA UTILIZING NEURAL NETWORK 412
DISPLAY PROCESSED DATA AS ELECTROPHEROGRAM IN DISPLAY DEVICE 414

FIG. 4

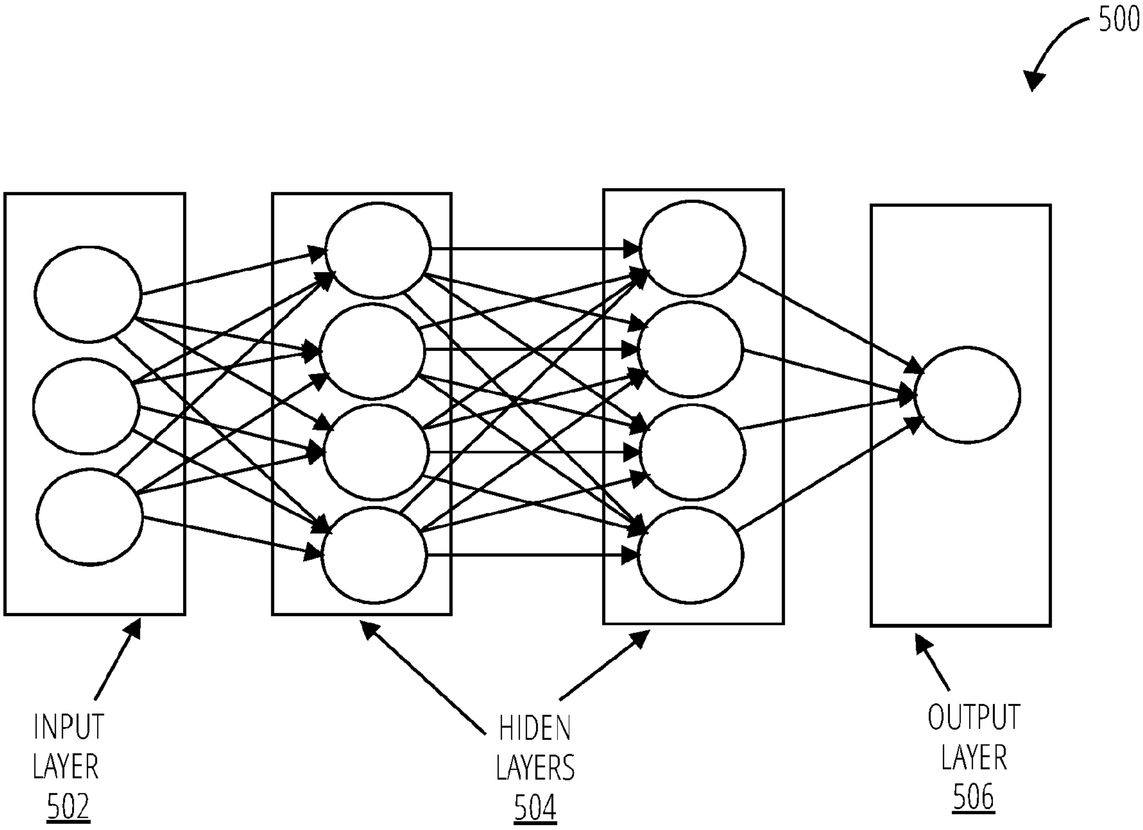


FIG. 5

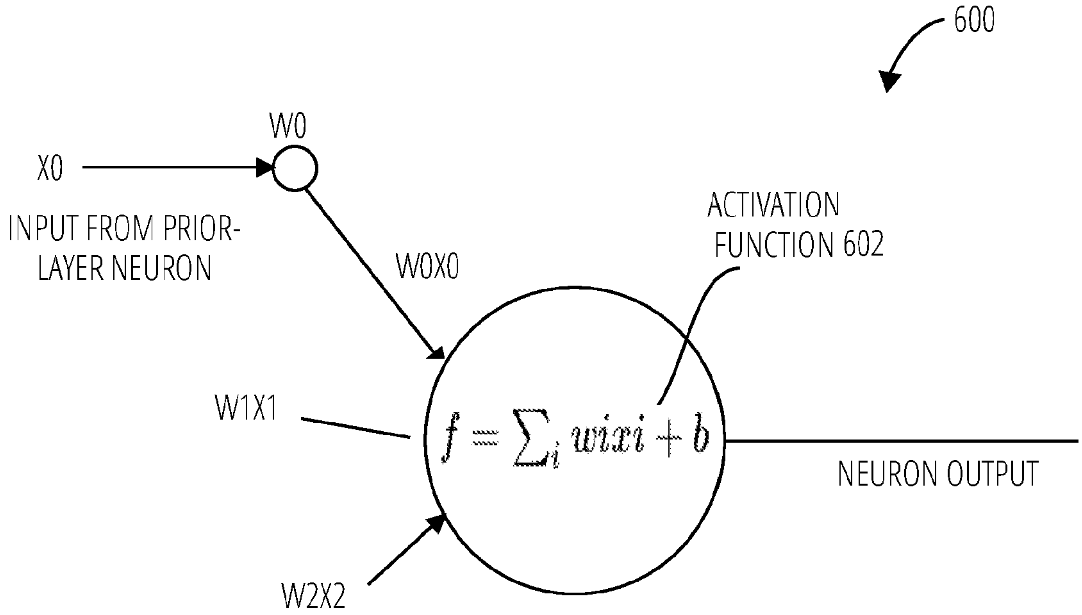
600
W0
X0
INPUT FROM PRIOR-
LAYER NEURON
W0X0
ACTIVATION
FUNCTION 602
W1X1
$f = \sum_i wixi + b$
NEURON OUTPUT
W2X2

FIG. 6

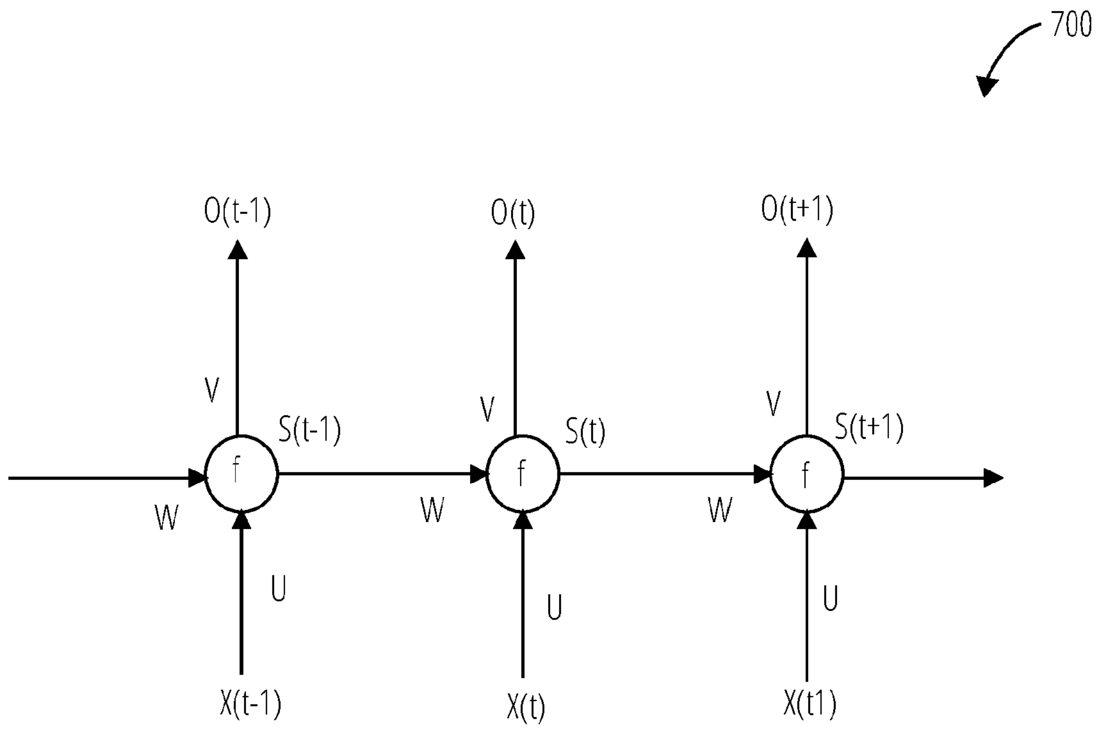


FIG. 7

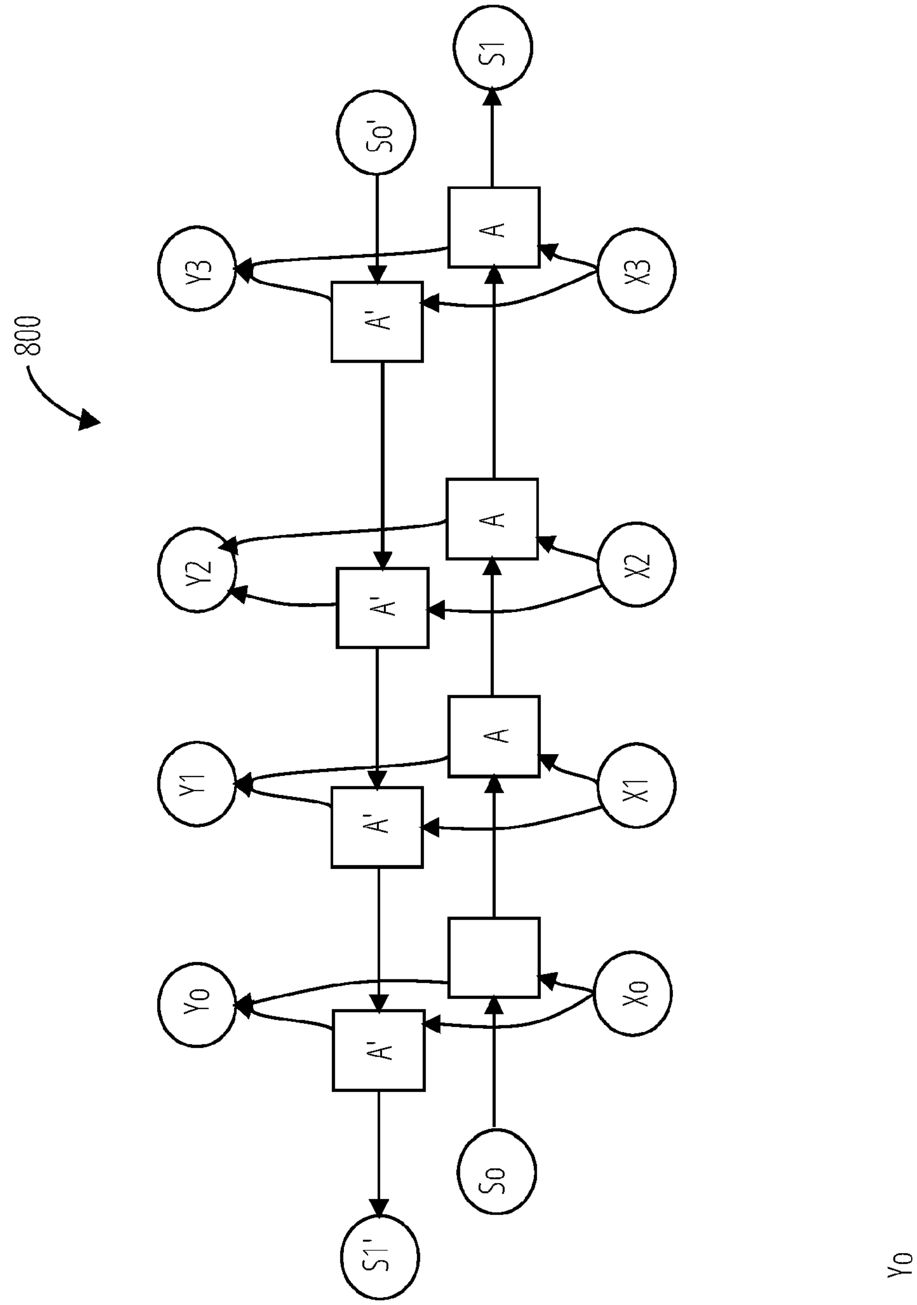


FIG. 8

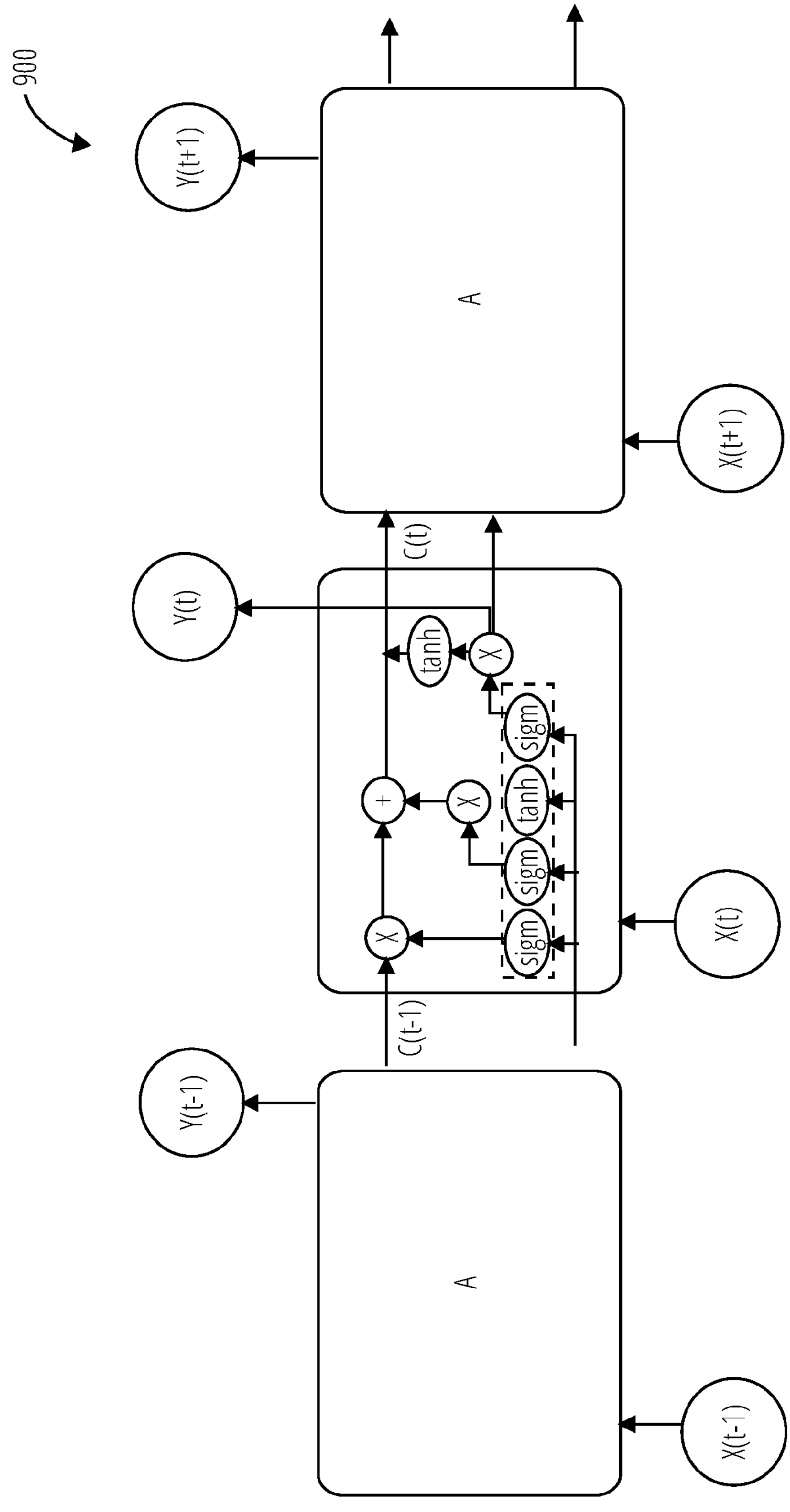


FIG. 9

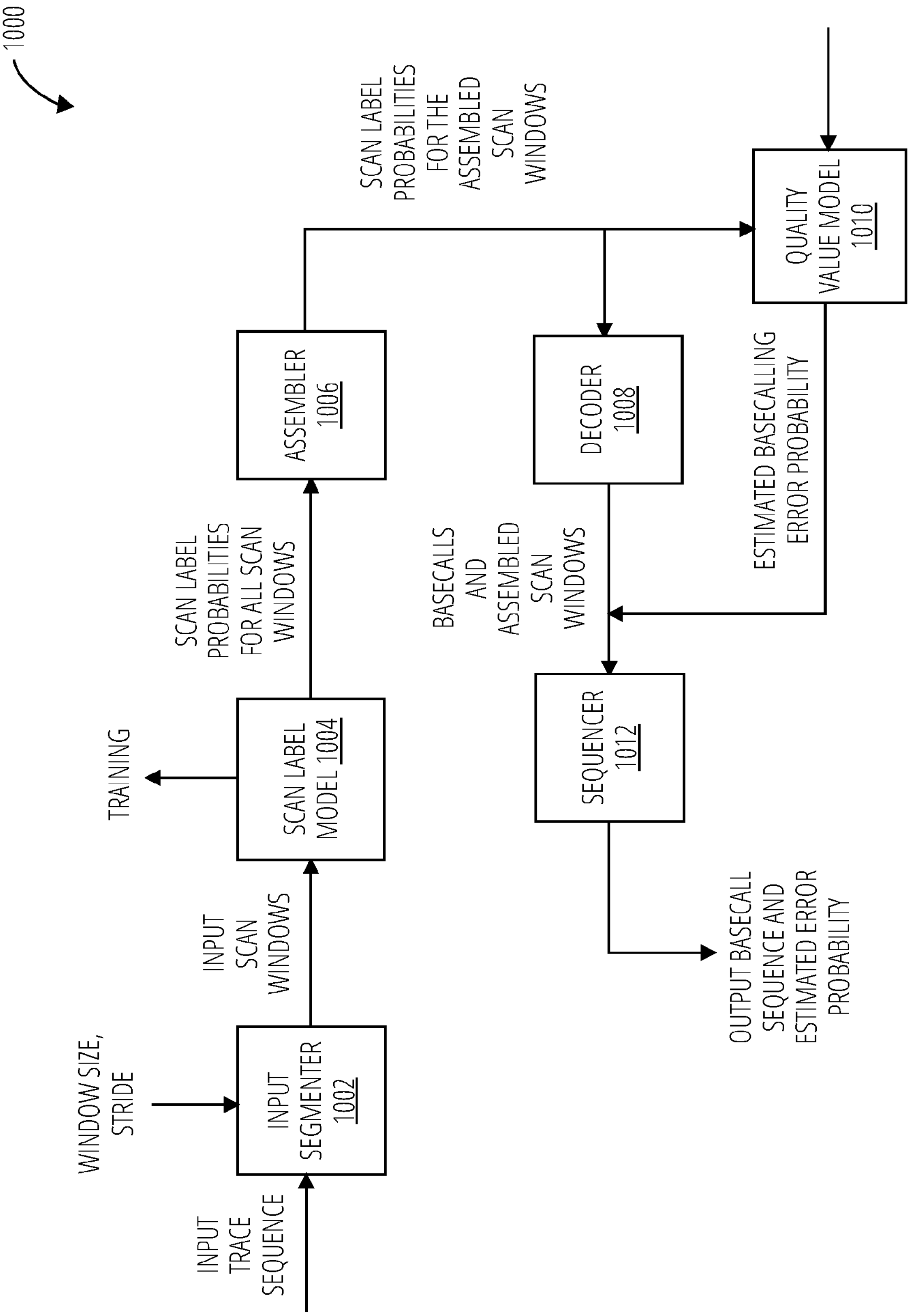
1000
INPUT TRACE SEQUENCE
WINDOW SIZE, STRIDE
INPUT SEGMENTER 1002
INPUT SCAN WINDOWS
SCAN LABEL MODEL 1004
TRAINING
SCAN LABEL PROBABILITIES FOR ALL SCAN WINDOWS
ASSEMBLER 1006
SCAN LABEL PROBABILITIES FOR THE ASSEMBLED SCAN WINDOWS
DECODER 1008
BASECALLS AND ASSEMBLED SCAN WINDOWS
QUALITY VALUE MODEL 1010
ESTIMATED BASECALLING ERROR PROBABILITY
SEQUENCER 1012
OUTPUT BASECALL SEQUENCE AND ESTIMATED ERROR PROBABILITY

FIG. 10

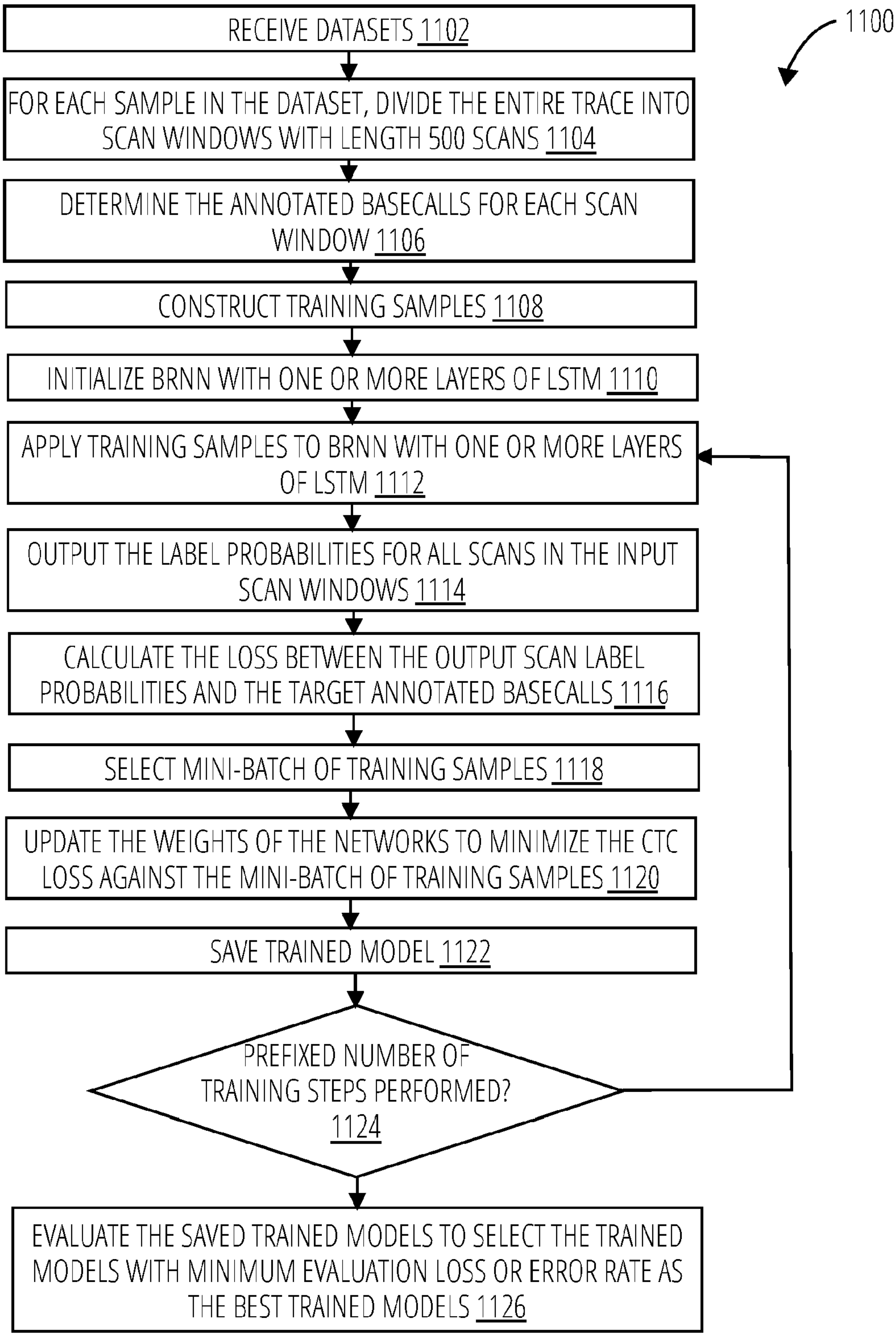
1100
RECEIVE DATASETS 1102
FOR EACH SAMPLE IN THE DATASET, DIVIDE THE ENTIRE TRACE INTO SCAN WINDOWS WITH LENGTH 500 SCANS 1104
DETERMINE THE ANNOTATED BASECALLS FOR EACH SCAN WINDOW 1106
CONSTRUCT TRAINING SAMPLES 1108
INITIALIZE BRNN WITH ONE OR MORE LAYERS OF LSTM 1110
APPLY TRAINING SAMPLES TO BRNN WITH ONE OR MORE LAYERS OF LSTM 1112
OUTPUT THE LABEL PROBABILITIES FOR ALL SCANS IN THE INPUT SCAN WINDOWS 1114
CALCULATE THE LOSS BETWEEN THE OUTPUT SCAN LABEL PROBABILITIES AND THE TARGET ANNOTATED BASECALLS 1116
SELECT MINI-BATCH OF TRAINING SAMPLES 1118
UPDATE THE WEIGHTS OF THE NETWORKS TO MINIMIZE THE CTC LOSS AGAINST THE MINI-BATCH OF TRAINING SAMPLES 1120
SAVE TRAINED MODEL 1122
PREFIXED NUMBER OF TRAINING STEPS PERFORMED? 1124
EVALUATE THE SAVED TRAINED MODELS TO SELECT THE TRAINED MODELS WITH MINIMUM EVALUATION LOSS OR ERROR RATE AS THE BEST TRAINED MODELS 1126

FIG. 11

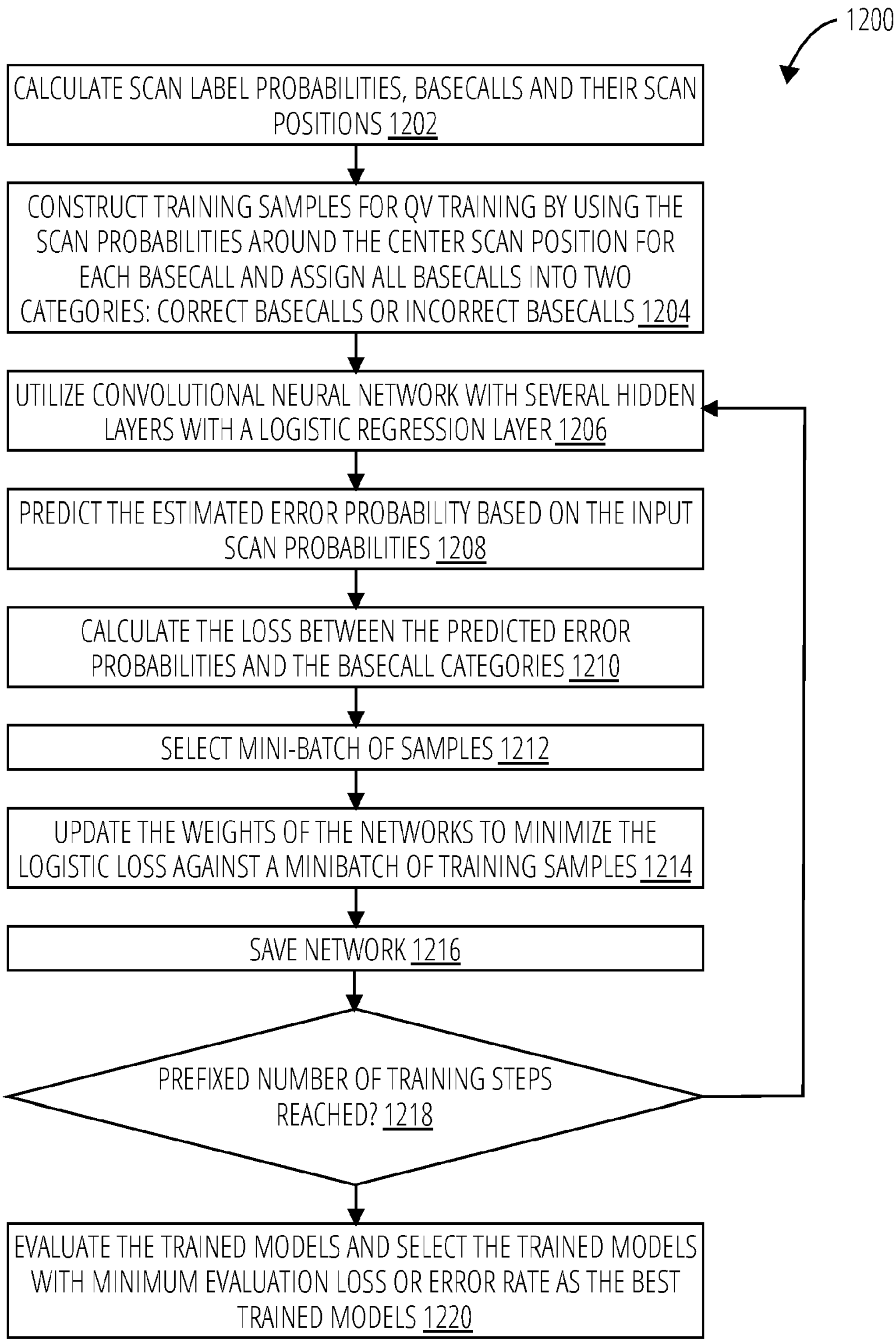
1200
CALCULATE SCAN LABEL PROBABILITIES, BASECALLS AND THEIR SCAN POSITIONS 1202
CONSTRUCT TRAINING SAMPLES FOR QV TRAINING BY USING THE SCAN PROBABILITIES AROUND THE CENTER SCAN POSITION FOR EACH BASECALL AND ASSIGN ALL BASECALLS INTO TWO CATEGORIES: CORRECT BASECALLS OR INCORRECT BASECALLS 1204
UTILIZE CONVOLUTIONAL NEURAL NETWORK WITH SEVERAL HIDDEN LAYERS WITH A LOGISTIC REGRESSION LAYER 1206
PREDICT THE ESTIMATED ERROR PROBABILITY BASED ON THE INPUT SCAN PROBABILITIES 1208
CALCULATE THE LOSS BETWEEN THE PREDICTED ERROR PROBABILITIES AND THE BASECALL CATEGORIES 1210
SELECT MINI-BATCH OF SAMPLES 1212
UPDATE THE WEIGHTS OF THE NETWORKS TO MINIMIZE THE LOGISTIC LOSS AGAINST A MINIBATCH OF TRAINING SAMPLES 1214
SAVE NETWORK 1216
PREFIXED NUMBER OF TRAINING STEPS REACHED? 1218
EVALUATE THE TRAINED MODELS AND SELECT THE TRAINED MODELS WITH MINIMUM EVALUATION LOSS OR ERROR RATE AS THE BEST TRAINED MODELS 1220

**FIG. 12**

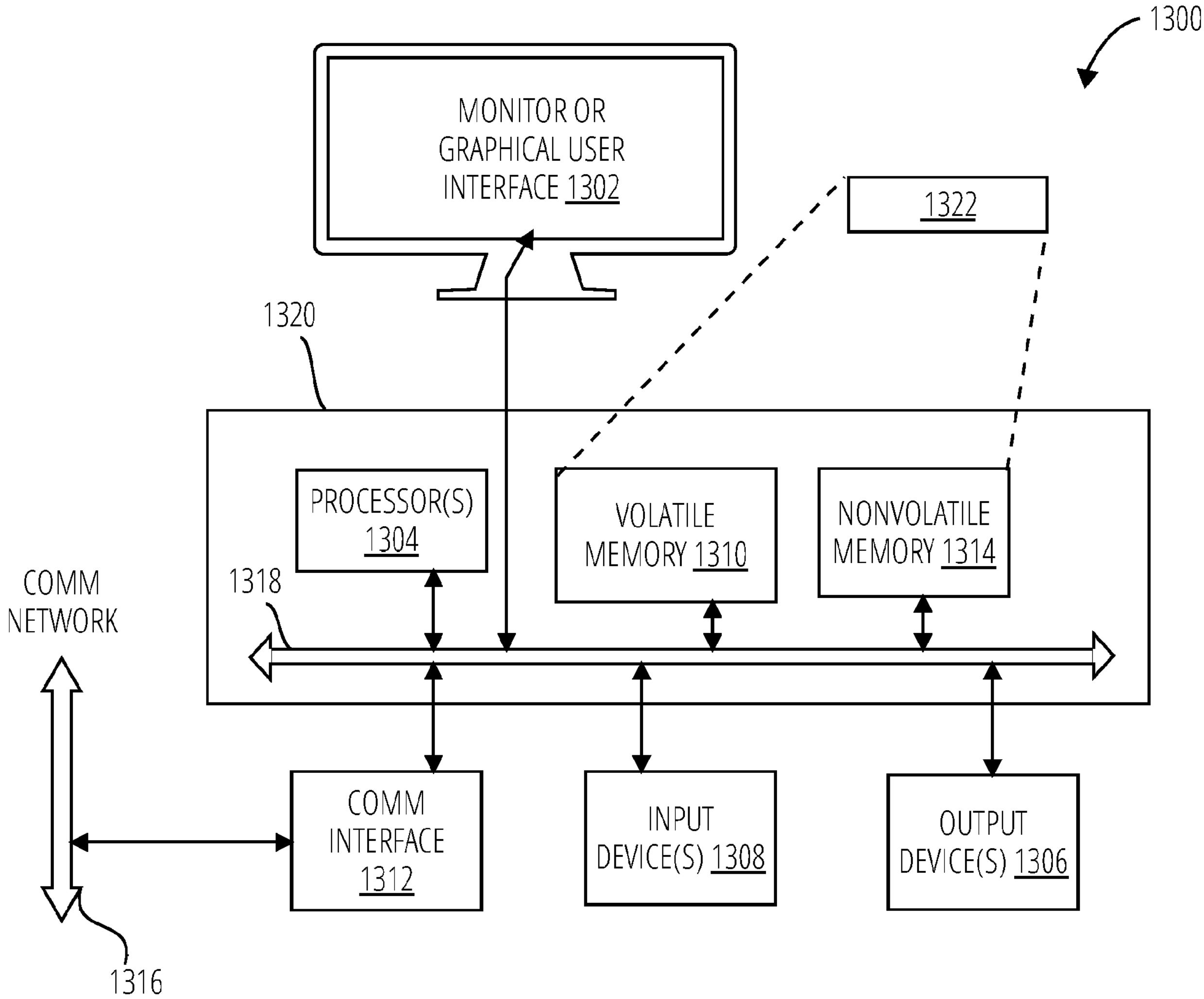
1300
MONITOR OR
GRAPHICAL USER
INTERFACE 1302
1322
1320
PROCESSOR(S)
1304
VOLATILE
MEMORY 1310
NONVOLATILE
MEMORY 1314
COMM
NETWORK
1318
COMM
INTERFACE
1312
INPUT
DEVICE(S) 1308
OUTPUT
DEVICE(S) 1306
1316

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **OMNIYAH G. MOHAMMED**. Novel algorithms for accurate DNA base-calling. *JOURNAL OF BIOMEDICAL SCIENCE AND ENGINEERING*, 01 January 2013, vol. 6 (02), ISSN 1937-6871, 165-174
- **HAOTIAN TENG**. Chiron: translating nanopore raw signal directly into nucleotide sequence using deep learning. *GIGASCIENCE*, 10 April 2018, vol. 7 (5)
- **RATKOVIC M**. RATKOVIC MDeep learning Model for Base Calling of MinION Nanopore Reads. *INTERNET CITATION*, 01 June 2017, 1-48